# EUROPEAN PATENT SPECIFICATION

(11) **EP 1 563 302 B1**
(45) Date of publication and mention of the grant of the patent: **14.09.2011**
(21) Application number: 03736436.1
(22) Date of filing: 13.02.2003
(51) Int. Cl.: G01N 33/574

(54) **METHODS FOR SELECTING TREATMENT REGIMENS AND PREDICTING OUTCOMES IN CANCER PATIENTS**
VERFAHREN ZUR AUSWAHL VON BEHANDLUNGSSCHEMATA UND VORHERSAGE VON BEHANDLUNGSRESULTATEN BEI KREBSPATIENTEN
PROCEDES POUR SELECTIONNER DES REGIMES DE TRAITEMENT ET POUR PREDIRE DES RESULTATS CHEZ DES PATIENTS ATTEINTS DE CANCER

(30) Priority: 13.02.2002 US 356928 P; 09.08.2002 US 402311 P
(43) Date of publication of application: 17.08.2005
(60) Divisional application: 10181640.3
(73) Proprietor: AMERICAN DIAGNOSTICA, INC., Stamford, CT 06911-0215 (US)
(72) Inventor: HARBECK, Nadia, 83624 Otterfing (DE); KATES, Ronald, E., 83624 Otterfing (DE); SCHMITT, Manfred, 81669 München (DE); FOEKENS, John, A., 2908 XA Capelle aan de IJssel (NL)
(74) Representative: Jones Day
(86) International application number: PCT/US2003/004538
(87) International publication number: WO 2003/082072

(56) References cited:
- WO-A-03/073910
- JAENICKE FRITZ ET AL: "Randomized adjuvant chemotherapy trial in high-risk, lymph node-negative breast cancer patients identified by urokinase-type plasminogen activator and plasminogen activator inhibitor type 1" JOURNAL OF THE NATIONAL CANCER INSTITUTE (BETHESDA), vol. 93, no. 12, 20 June 2001 (2001-06-20), pages 913-920, XP002456092 ISSN: 0027-8874
- LOOK M P ET AL: "Pooled analysis of prognostic impact of urokinase-type plasminogen activator and its inhibitor PAI-1 in 8377 breast cancer patients" JOURNAL OF THE NATIONAL CANCER INSTITUTE, US DEPT. OF HEALTH, EDICATIONAND WELFARE, PUBLIC HEALTH, US, vol. 94, no. 2, 16 January 2002 (2002-01-16), pages 116-128, XP002386409 ISSN: 0027-8874
- HARBECK NADIA ET AL: "Invasion marker PAI-1 remains a strong prognostic factor after long-term follow-up both for primary breast cancer and following first relapse" BREAST CANCER RESEARCH AND TREATMENT, vol. 54, no. 2, March 1999 (1999-03), pages 147-157, XP002456093 ISSN: 0167-6806
- FOEKENS JOHN A ET AL: "Urokinase-type plasminogen activator and its inhibitor PAI-1: Predictors of poor response to tamoxifen therapy in recurrent breast cancer" JOURNAL OF THE NATIONAL CANCER INSTITUTE (BETHESDA), vol. 87, no. 10, 1995, pages 751-756, XP009091133 ISSN: 0027-8874
- HARBECK NADIA ET AL: "Enhanced benefit from adjuvant chemotherapy in breast cancer patients classified high-risk according to urokinase-type plasminogen activator (uPA) and plasminogen activator inhibitor type 1 (n = 3424)" CANCER RESEARCH, AMERICAN ASSOCIATION FOR CANCER RESEARCH, BALTIMORE, MD, US, vol. 62, no. 16, 15 August 2002 (2002-08-15), pages 4617-4622, XP002419683 ISSN: 0008-5472
- HARBECK NADIA ET AL: "Clinical relevance of invasion factors urokinase-type plasminogen activator and plasminogen activator inhibitor type 1 for individualized therapy decisions in primary breast cancer is greatest when used in combination." JOURNAL OF CLINICAL ONCOLOGY : OFFICIAL JOURNAL OF THE AMERICAN SOCIETY OF CLINICAL ONCOLOGY 15 FEB 2002, vol. 20, no. 4, 15 February 2002 (2002-02-15), pages 1000-1007, XP002456094 ISSN: 0732-183X
- FOX: 'Plaminogen activator inhibitor-1 as a measure of vascular remodelling in breast cancer' JOURNAL OF PATHOLOGY vol. 195, September 2001, pages 236 - 243, XP008046551
- FOEKENS J A ET AL: "The urokinase system of plasminogen activation and prognosis in 2780 breast cancer patients", CANCER RESEARCH, AMERICAN ASSOCIATION FOR CANCER REREARCH, US, vol. 60, no. 3, 1 February 2000 (2000-02-01), pages 636-643, XP002386408, ISSN: 0008-5472

## Description

### 1. FIELD OF THE INVENTION

The invention relates generally to the field of cancer prognosis, treatment selection, and treatment outcome prediction. More particularly, the present invention relates to methods for selecting a treatment protocol for a subject based on at least two prognostic factors for cancer, particularly breast cancer, leukemia, and plasmacytoma. The factors include urokinase-type plasminogen activator (uPA) and its inhibitor plasminogen activator inhibitor-1 (PAI-1). The pre77sent invention provides methods comprising measuring the levels of uPA and PAI-1 or mRNA encoding uPA and PAI-1 in cancer tissue from a cancer patient and selecting a treatment regimen for cancer. The selection of treatment regimen is based upon uPA/PAI-1 levels or levels of mRNA encoding uPA and PAI-1. Also, methods to predict the highest expected benefit, i.e., disease-free and/or overall survival in patients with or without a particular treatment are provided.

### 2. BACKGROUND OF THE INVENTION

Cancer is characterized primarily by an increase in the number of abnormal cells derived from a given normal tissue, invasion of adjacent tissues by these abnormal cells, and lymphatic or blood-borne spread of malignant cells to regional lymph nodes and to distant sites (metastasis). Clinical data and molecular biological studies indicate that cancer is a multistep process that begins with minor preneoplastic changes, which may under certain conditions progress to neoplasia.

Pre-malignant abnormal cell growth is exemplified by hyperplasia, metaplasia, or most particularly, dysplasia (for review of such abnormal growth conditions, *see* Robbins & Angell, 1976, Basic Pathology, 2d Ed., W.B. Saunders Co., Philadelphia, pp. 68-79.) The neoplastic lesion may evolve clonally and develop an increasing capacity for growth, metastasis, and heterogeneity, especially under conditions in which the neoplastic cells escape the host's immune surveillance (Roitt, I., Brostoff, J. and Kale, D., 1993, Immunology, 3rd ed., Mosby, St. Louis, pps. 17.1-17.12). The plasminogen activator system plays a key role in tumor invasion and metastasis (Andreasen, et al., 1997, Int. Journal Cancer 72: 1-22; Schmitt, et al., 1997, Thrombosis Haemostasis 78: 285-296). A critical balance of urokinase-type plasminogen activator (uPA), its cell surface receptor uPA-R (CD 87), and its inhibitor, plasminogen activator inhibitor-1 (PAI-1) is the prerequisite for efficient focal proteolysis, adhesion and migration, and hence, subsequent tumor cell invasion and metastasis.

In clinical practice, accurate diagnosis of various subtypes of cancer is important because treatment options, prognosis, and the likelihood of therapeutic response all vary broadly depending on the diagnosis. Accurate prognosis, or determination of distant metastasis-free survival or overall survival could allow the oncologist and the patient to make treatment decisions. Furthermore, accurate prediction of poor prognosis would greatly impact clinical trials for new breast cancer therapies, because potential study patients could then be stratified according to prognosis. Trials could then be limited to patients having poor prognosis, in turn making it easier to discern if an experimental therapy is efficacious.

The incidence of breast cancer, a leading cause of death in women, has been gradually increasing in the United States over the last thirty years. Its cumulative risk is relatively high, 1 in 8 women, for example, by age 85 in the United States. In fact, breast cancer is the most common cancer in women and the second most common cause of cancer death in the United States. In 1997, it was estimated that 181,000 new cases were reported in the U.S., and that 44,000 people would die of breast cancer (Parker et al., 1997, CA Cancer J Clin. 47 : 5; Chu et al., 1996, J. Nat. Cancer Inst. 88:1571).

Breast cancer arises from a malignancy of epithelial cells in the female, and occasionally the male, usually of adenocarcinoma origin initiated in the ductal breast epithelium. Breast Cancer is the most common non-dermal malignancy in women and 192,200 cases are anticipated in the U.S. for the upcoming year. Despite recent advances in early diagnosis and treatment, 40,200 U.S. women have succumbed to this disease in the year 2000 (Greenlee et al., 2001, Cancer Statistics 51(1):15).

A marker-based approach to tumor identification and characterization promises improved diagnostic and prognostic reliability. Typically, the diagnosis of breast cancer and other types of cancer requires histopathological proof of the presence of the tumor. In addition to diagnosis, histopathological examinations also provide information about prognosis and selection of treatment regimens. Prognosis may also be established based upon clinical parameters such as tumor size, tumor grade, the age of the patient, and lymph node metastasis.

With the available and potent conventional drug regimens as well as the advent of novel therapy approaches targeting specific biological pathways, the determination of optimal treatment of primary breast cancer is becoming increasingly complex. The outcome of a treatment of a patient with cancer is often unpredictable. Only a portion of the patients respond to a certain type of treatment. The patients receiving a specific type of treatment are subj ected to an unnecessary suffering since adverse reactions often are obtained from certain treatment used. Some treatments elicit more severe reaction from the patient than other treatments. Mostly, the effect of a treatment is not shown until 3-6 months after treatment. It would therefore be of great importance if patients with a high probability to respond could be identified before the onset of treatment. To date, no set of satisfactory predictors for prognosis based on the clinical information alone has been identified.

Currently, about 50% of the patients with primary breast cancer do not have auxillary lymph node involvement, and this percentage is increasing (Hellman et al., 2000, Diseases of the breast, 2nd ed., Philadelphia; p. 407-23; Clark et al., 1988, Semin Oncol 15(2 Suppl 1):20-5.). It is not possible to identify reliably the low-risk patients (who can be spared adjuvant chemotherapy) by traditional histomorphologic and clinical characteristics, such as tumor size, histologic grade, age, steroid hormone receptor status, or menopausal status. McGuire et al., 1992, N Engl J Med 326:1756-61. If these characteristics were used to select therapies for patients, as recommended by the 1998 and 2001 St. Gallen consensus statements (Zujewski et al., 1998, J Natl Cancer Inst 90:1587-9; 7th International Consensus Conference on Adjuvant Therapy of Primary Breast Cancer, St. Gallen, Switzerland, February 2001), up to 90% of the patients with lymph node-negative breast cancer would be candidates for adjuvant chemotherapy, although only about 30% of the patients with lymph node-negative breast cancer will relapse and thus need adjuvant chemotherapy. This discrepancy has prompted a search for additional prognostic factors.

Cutoffs of 3ng uPa / mg protein and 14ng PAI-1 / mg protein have been used to distinguish between high and low antigen levels in breast cancer tissue. Using uPA and PAI-1 it was possible to dichotomize breast cancer patients into subpopulations of high and low risk and to take decisions which therapy regimen to apply for (Harbeck et al, Breast Cancer Res & Treatment vol. 54, 1999, p. 147 - 247 and Jänicke et al, J. Natl. Cancer Inst. vol. 93, no. 12, 2001, p. 913 - 920). Look et al, J. Natl. Cancer Inst., vol. 94, no. 2, January 2002, p. 116 - 128, present a retrospective pooled analysis of uPa and PAI-1 as prognostic factor in breast cancer patients.

It would, therefore, be beneficial to provide specific methods for selecting treatment regimen in a cancer subject, in particular, breast cancer, leukemia and plasmacytoma. The purpose of the present invention is to provide a method of predicting a response to a treatment regimen for a subject based on the levels of uPA and PAI-1 or mRNA encoding uPA and PAI-1. This method identify subjects that belong to a high risk group and a low risk group for recurrence of cancer in particular breast cancer, leukemia and plasmacytoma, and predict disease-free and overall survival under certain treatment regimens. Thus, appropriate treatment regimen may be implemented for each group. The present invention also provides a method of predicting a response to a treatment regimen for a subject based on the levels of uPA and PAI-1 or mRNA encoding uPA and PAI-1. In the case of solid tumors or breast cancer, the method further based on the number of lymph nodes that are affected.

### 3. SUMMARY OF THE INVENTION

The present invention is based upon the observation of the present inventors that when the level of the prognostic factors, urokinase-type plasminogen activator (uPA) and its inhibitor plasminogen activator inhibitor-1 (PAI-1), are assayed in the tumors of breast cancer patients, a high level, (*i.e*., over a specified "cut-off value") of either one or both of the two factors indicates that the patients are high-risk breast cancer patients, *i.e*., they have an increased risk, in particular, for early relapse. These patients benefitted significantly from aggressive therapy, such as adjuvant systemic chemotherapy, after the initial surgery to remove the tumor tissue. Those patients having low levels (*i.e*., below a specific "cut-off value") of both uPA and PAI-1 could be classified as "low risk" *i.e.,* having a low risk of relapse and did not significantly benefit from aggressive therapy in view of the low survival benefit of these treatments in "low risk" patients weighed against possible adverse health effects of the aggressive treatment, *i.e*., "total patient benefit from therapy". Conversely, among patients who, according to other criteria or classification methods, might have been classified as "low risk", high levels of either or both of said factors may indicate a significant expected benefit, *i.e*., reduction of relapse risk, of aggressive therapy sufficient to outweigh adverse health effects of the treatments in question. The present invention relates to a method for selecting from two or more treatment regimens, a treatment regimen having the highest expected benefit to a patient with primary breast cancer or, a method for predicting an expected benefit in a comparable population of breast cancer patients by selecting from two or more treatment regimens, a treatment regimen having the highest expected benefit, said patient having primary tumor tissue, said method comprising:
(a) measuring the level of uPA and the level of PAI-1 or mRNA encoding uPA and mRNA encoding PAI-1 in a sample of said primary tumor tissue of said patient;
(b) classifying said patient as low risk if the level of uPA is lower than a uPA cut-off value of at least the 55^{th} percentile and at most the 75^{th} percentile of normalized uPA levels in a randomized population of breast cancer patients and the level of PAI-1 is lower than a PAI-1 cut-off value of at least the 61^{st} percentile and at most the 81^{st} percentile of normalized PAI-1 levels in a randomized population of breast cancer patients, or as high risk if either the level of uPA is higher than the uPA cut-off value or the level of PAI-1 is higher than the PAI-1 cut-off value;
(c) if said patient is classified as low risk in step (b), selecting a treatment regimen from said two or more treatment regimens that results in the highest expected benefit in a comparable population of low risk cancer patients; and
(d) if said patient is classified as high risk in step (b), selecting a treatment regimen from said two or more treatment regimens that results in the highest expected benefit in a comparable population of high risk cancer patients.

The present invention relates to methods for selecting a treatment regimen beyond surgical removal of tumor tissue for any breast cancer subject, including subjects who have detectable cancer cells in lymph node tissue (*i.e*., "node positive patients," having cancer cells detected in 1, 2, 3, 4, 5, 6, 7, 8, 9, 10 or more lymph nodes) and subjects who do not have detectable cancer cells in lymph node tissue (*i.e*., "node negative patients").

The method comprises measuring the levels ofuPA and PAI-1 or mRNA encoding uPA and PAI-1 in a subject, preferably cancer tissue from the subject (e.g., collected by surgery) or a tissue sample comprising cancer cells, (e.g., collected by core needle biopsy or body fluid aspiration); and, utilizing these values, classifying the subject as "low risk" or "high risk" and selecting a treatment having the greatest expected benefit, which includes disease-free survival, particularly long term disease-free survival and/or overall survival, for a comparable population. In particular embodiments, the method of the invention is used to determine whether a subject should undergo an aggressive treatment regimen or a non-aggressive treatment regimen based upon the expected benefit outcomes of subjects in the same classification, *i.e*., low or high risk, in a comparable population. In a particular embodiment, the method is used to determine whether to administer a treatment regimen other than CMF chemotherapy.

"Expected benefit" is defined as the average demonstrated overall survival and/or disease-free survival (including long term disease-free survival) balanced by the negative effect on the quality of life due to the side effects of a particular cancer treatment.

A "comparable population" is defined as a population that shares clinically relevant factors, such as, but not limited to, number of lymph nodes affected (nodal status), tumor size, tumor grade, patient's age, hormone receptor status, menopausal status, other tumor biological factors (*e.g*., Her-2 expression), and any other factors that one skilled in the art considers in classifying cancer patients.

"Long term disease-free survival" is defined as a disease-free status or lack of recurrence of the breast cancer for a period of over 3, 5, 6, 8, 10, 12, 15, or 30 years or more. Long term overall survival is defined as a patient surviving for a period of over 3, 5, 6, 8, 10, 12, 15, or 30 years or more after the patient is diagnosed with cancer.

High risk subjects are identified by high levels of both uPA and PAI-1 determined by cut-off values for these indicators. High risk subjects are identified by high levels of both mRNA encoding uPA and PAI-1 determined by cut-off values for these indicators. High risk subjects (particularly, high risk node-positive subjects) may have 4 or more affected lymph nodes. In a specific embodiment, uPA and PAI-1 levels may be measured by the antigen levels in primary tumor tissue extracts. In a preferred embodiment, the levels of uPA and PAI-1 or mRNA encoding uPA and PAI-1 are measured by any assay method. In a preferred embodiment, the mRNA encoding uPA and PAI-1 are measured by RT-PCR amplification. In other preferred embodiments, the RT-PCR amplification is performed on paraffin sections of a patient sample or one or more single cells of said patient sample. The patient sample comprises one or more cancer cells. In a specific embodiment, a high level of uPA or mRNA encoding uPA corresponds to levels above a cut-off value of at least about the 55^{th} percentile and no more than about the 75^{th} percentile of normalized (i.e., adjusted for differences in measured values due to differences in assay methods) uPA levels or levels of mRNA encoding uPA for a randomized group of patients using any assay. In specific embodiments, a high level of uPA or mRNA encoding uPA corresponds to levels above a cut-off value of at least about the 60^{th}, 65^{th}, or 70^{th} percentile. In a specific embodiment, a high level of PAI-1 or mRNA encoding PAI-1 corresponds to a PAI-1 levels above a cut-off value of at least about the 61^{st} percentile and no more than about the 81^{st} percentile of normalized PAI-1 levels for a randomized group of patients using any assay. In specific embodiment, a high level of PAI-1 or mRNA encoding PAI-1 corresponds to a PAI-1 level above a cut-off value of at least about the 65^{th}, 70^{th}, or 75^{th} percentile of normalized PAI-1 levels.

Low risk subjects are identified by low levels of both uPA and PAI-1 or mRNA encoding uPA and PAI-1 *i.e.,* below the values determined as the "cutoff' values for uPA and PAI-1 or mRNA encoding uPA and PAI-1. Low risk subjects are also idenfied as node-negative patients having low levels of both uPA and PAI-1. Low risk node-positive subjects may have 3 or less affected lymph nodes and having low levels of both uPA and PAI-1. In a preferred embodiment, the mRNA encoding uPA and PAI-1 are measured by RT-PCR amplification. In preferred embodiments, the RT-PCR amplification is performed on parafin sections of a patient sample or one or more single cells of said patient sample. The patient sample comprises one or more cancer cells. In a specific embodiment, a low level of uPA or mRNA encoding uPA corresponds to levels below the cut-off value of at least about the 55^{th} percentile and no more than about the 75^{th} percentile of normalized uPA levels for a randomized group of patients using any assay. In specific embodiments, a low level of uPA or RNA encoding uPA corresponds to levels below the cut-off value of at least about the 60^{th}, 65^{th}, or 70^{th} percentile. In specific embodiment, a low level of PAI-1 or mRNA encoding PAI-1 corresponds to a PAI-1 levels below a cut-off value of at least about the 65^{th}, 70^{th}, 75^{th} percentile of normalized PAI-1 levels. In specific embodiments, a low level of PAI-1 or mRNA encoding PAI-1 corresponds to levels below a cut-off value of about the 65^{th}, 70^{th}, or 75^{th} percentile.

Aggressive post-surgery treatment regimens are treatment regimens that have significant side-effects. These treatment regimens may include, but are not limited to, chemotherapy, adjuvant chemotherapy, adjuvant CMF chemotherapy, adjuvant non-CMF chemotherapy, adjuvant anthracyclin-containing chemotherapy, and adjuvant taxane-containing chemotherapy, and may include adjuvant endocrine therapy, including for example, anti-estrogens, aromatase inhibitors, gestagens, and also includes radiation therapy, or gene therapy. Although these treatment regimens are usually selected for high risk patients, certain aggressive treatments may be very effective even in low risk patients.

Non-aggressive post-surgery treatment regimens are treatment regimens that have less significant side-effects. These treatment regimens may include, but are not limited to, non-treatment, radiation therapy and adjuvant endocrine therapy, such as, anti-estrogens (e.g., tamoxifen therapy), aromatase inhibitors, gestagens, immunotherapy, and tumor-biological therapy, *e.g*. HERCEPTIN®, anti-uPA therapies, including anti-uPA and anti-PAI-1 monoclonal antibodies, and uPA (and uPA receptor) and PAI-1 peptides and small molecule inhibitors. Although these treatment regimens are usually selected for low risk patients, certain non-aggressive treatments may be very effective and even significantly efficacious in high risk patients.

The present invention relates to methods for predicting an expected benefit in a comparable population of breast cancer patients by providing chemotherapy over hormone therapy for breast cancer patients that are classified as high risk by measuring the uPA, PAI-1 or uPA and PAI-1 levels or the levels of mRNA encoding uPA and PAI-1. Other clinical factors for classifying breast cancer patients may be used in conjunction with these two factors.

The present invention relates to methods for predicting an expected benefit in a comparable population of cancer patients by selecting a cancer patient for preventive treatment for relapse of cancer subsequent to administration of a first treatment regimen. In preferred embodiments, the cancer is breast cancer, leukemia or plasmacytoma. The method comprises measuring uPA, PAI-1, or, uPA and PAI-1 levels or the levels of mRNA encoding uPA and PAI-1 in the cancer patients or a tissue sample of the cancer patient; classifying the patients as low or high risk based upon the uPA, PAI-1, or uPA and PAI-1 levels or the levels of mRNA encoding uPA and PAI-1; and selecting one or more high risk subjects for a first treatment regimen. In a specific embodiment, the sample is obtained from a primary tumor of the cancer patient. Subsequently, patients that are classified as high risk for cancer relapse are further treated by a preventive treatment. The preventive treatment selected is based on the specific relapse site. In a specific embodiment, the relapse occurs in bone. In a specific embodiment, the preventive treatment comprises administration of bisphosphonate drugs to the patient.

The present invention also relates to a method for predicting an expected benefit in a comparable population of breast cancer patients after removal of primary tumor tissue. The method comprises measuring the level ofuPA and PAI-1 or levels ofmRNA encoding uPA and PAI-1 in said primary tumor tissue of said patient. The patient is classified as low risk if PAI-1 levels is low. Low level of PAI-1 corresponds to levels below a cut-off value of at least about the 61^{st} percentile and no more than about the 81^{st} percentile of normalized PAI-1 levels for a randomized group of patients using any assay. In specific embodiments, a low level of uPA corresponds to levels below the cut-off value of at least about the 60^{th}, 65^{th}, or 70^{th} percentile. In specific embodiments, a low level of PAI-1 corresponds to levels below a cut-off value of about the 65^{th}, 70^{th}, or 75^{th} percentile. A patient is classified as high risk if the level of PAI-1 is high. The patient is classified as high risk if the level of PAI-1 corresponds to above a cut-off value of at least about the 61^{st} percentile and no more than about the 81^{st} percentile of normalized PAI-1 levels for a randomized group of patients using any assay. In preferred embodiments, the levels of mRNA encoding uPA and PAI-1 are measured by RT-PCR amplification. In specific embodiments, a high level of PAI-1 corresponds to levels above a cut-off value of at least about 65^{th}, 70^{th}, or 75^{th} percentile. If the patient is classified as low risk, a non-aggressive treatment regimen is selected if that treatment regimen results in the highest expected benefit of treatment in a comparable population of low risk patients; and if the patient is classified as high risk, an aggressive treatment regimen is selected if that treatment regimen results in the highest expected benefit of treatment in a comparable population of high risk patients. In a specific embodiment, the aggressive treatment is chemotherapy.

The subject can be any animal, but, preferably, the subject is a mammal, and most preferably the subject is a human. In one embodiment, the method of the present invention is applicable to node-negative patients. In another embodiment, the method of the present invention is applicable to node-positive patients. In an embodiment, the method of the present invention is applicable to metastatic patients. In another embodiment, the method of the present invention is applicable to non-metastatic patients.

In one embodiment, the methods of the present invention include measuring nucleic acid molecules that encode the uPA and PAI-1 proteins or their naturally occurring variants that are indicative of uPA and PAI-1 expression. The methods of the present invention also encompass measuring uPA and PAI-1 gene products using antibodies directed against such uPA and PAI-1 gene products or conserved variants or fragments thereof. In one embodiment, the method employs antibodies directed against a fragment or other derivative of uPA and PAI-1 proteins which are at least 10 amino acids in length. In a specific embodiment, the method employs ELISA to measure the level of uPA and the level of PAI-1. In a more specific embodiment, the level of uPA and the level of PAI-1 are measured using Imubind #894 and Imubind #821 (American Diagnostica, Inc., Greenwich CT), respectively.

In a non-limiting example, nucleic acid molecules of uPA and PAI-1 can be used as diagnostic hybridization probes or as primers for quantitative RT- PCR analysis to determine expression levels of the uPA and PAI-1 gene products. In specific embodiment, the RT-PCR analysis is performed on paraffin sections of tissue sample of cancer patient or one or more single cells of said tissue sample. In specific embodiments, the tissue sample comprises one or more cancer cells.

Imaging methods, for imaging the localization and/or amounts of uPA and PAI-1 gene products in a patient, are also provided for diagnostic and prognostic use.

### 4. BRIEF DESCRIPTION OF THE FIGURES

FIGS. 1A & 1B. (A) The nucleotide sequence of uPA cDNA (SEQ ID NO:1). (B) The amino acid sequence of uPA (SEQ ID NO:2).
FIGS. 2A & 2B (A) The nucleotide sequence of PAI-1 cDNA (SEQ ID NO:3). (B) the amino acid sequence of PAI-1 (SEQ ID NO:4).
FIGS. 3A & 3B. (A) Enhanced risk group separation achieved by PAI-1 in low-uPA patients. (B) Enhanced risk group separation achieved by uPA in low-PAI-1 patients. Impact on disease-free survival (DFS) in *node-negative* breast cancer (no adjuvant systemic therapy).
FIG. 4. Prognostic impact of the four different combinations of uPA and PAI-1 on disease-free survival (DFS) in *node-negative* breast cancer (no adjuvant systemic therapy).
FIG. 5. Relative risk (RR) of recurrence associated with high uPA/PAI-1 in clinically relevant subgroups of *node-negative* breast cancer patients (without adjuvant systemic therapy).
FIGS. 6A & 6B. Impact of uPA/PAI-1 on disease-free survival (DFS) reflects effect of adjuvant systemic therapy *in primary* breast cancer patients.
FIG. 7. Benefits of therapy for different patient groups are illustrated in terms of relapse hazard ratios by levels of uPA/PAI (high vs. low) and by treatment groups (chemotherapy (CT) vs. hormone therapy (HT)). Ranges plotted include only standard errors in main effects.

### 5. DETAILED DESCRIPTION OF THE INVENTION

It is the observation of the present inventors that tumor levels of urokinase-type plasminogen activator (uPA) and of its inhibitor plasminogen activator inhibitor type 1 (PAI-1) are predictive factors for outcomes of lymph node-positive and lymph node-negative breast cancer patients. Patients with high levels of uPA and/or PAI-1 in their primary tumors, as defined by set cut-off values of uPA and PAI-1, had statistically significant shorter disease-free survival (DFS), including long-term disease-free survival, and overall survival (OS), including long term overall survival, than patients with low tumor levels for both uPA and Pay-1. In the present invention, the level of uPA and the level of PAI-1 or the levels of mRNA encoding uPA and PAI-1 in a patient are used to evaluate various treatment options, including no treatment, optionally, after removal of tumor tissue, in order to select a treatment regimen that provides benefit to a patient. In one aspect of the invention, the level of uPA and the level of PAI-1 or the levels of mRNA encoding uPA and PAI-1 in a patient, along with nodal status, are used to evaluate various treatment options in order to select a treatment regimen that provides optimal benefit to a patient. The benefit includes longer disease-free survival and overall survival after implementation of a selected treatment regimen as balanced by potential side effects of the treatment.

### 5.1. METHOD FOR SELECTING A TREATMENT REGIMEN

Adequate risk-group assessment for decisions on cancer therapy and prediction of response to treatment are prerequisites for individualized therapy designed for cancer patients. The methods of the present invention may be used to determine a treatment regimen by measuring the levels of uPA and PAI-1 of the levels of mRNA encoding uPA and PAI-1 in a subject. The clinical relevance of the two tumor invasion factors is greatest when used in combination, even though each factor alone may have predictive value for an expected benefit in a comparable population. The particular combination, uPA/PAI-1 is superior to either factor alone and supports risk-adapted individualized therapy decisions. These two factors strongly predict disease-free survival, including long-term disease-free survival, and overall survival in a population. Based upon the values, prediction of the length of disease-free survival and/or overall survival for the patient without treatment or with a particular treatment can be made. Furthermore, uPA/PAI-1 levels have a significant predictive impact on response to adjuvant chemotherapy. Hence, a more appropriate treatment regimen may be selected for the subject. Depending on whether the levels of uPA and/or PAI-1 or levels of mRNA encoding uPA and PAI-1 are above or below a set cut-off value, subjects maybe classified as high risk or low risk. High risk indicates that the subject may suffer from early relapse. For patients with a high probability of early relapse, further preventive treatment may be administered after a particular treatment has been administered. Low risk indicates that the subject has a low risk of relapse, thus, the subject may not significantly benefit from certain aggressive cancer treatments.

The patient may be classified as high risk or low risk depending on the level of uPA and level of PAI-1 or the levels of mRNA encoding uPA and PAI-1 measured as a percentile for a randomized group of cancer patients using one or more assays for uPA and PAI-1 or mRNA encoding uPA and PAI-1. A high level of uPA corresponds to levels that are higher than a cut-off level set at a value at least about the 55^{th} percentile and not more than about the 75^{th} percentile of normalized uPA levels for a randomized group of cancer patients using any assay. In specific embodiments, a high level of uPA corresponds to levels above a cut-off value of at least about the 60^{th}, 65^{th}, or 70^{th} percentile. In an embodiment, the cut-off value for uPA is at least about the 65^{th} percentile of normalized uPA levels for a randomized group of cancer patients using any assay. In another embodiment, the cut-off value for uPA is at least about the 70^{th} percentile of normalized uPA levels for a randomized group of cancer patients using any assay. A high level of PAI-1 corresponds to levels that are higher than a cut off level set at a value of at least about the 61^{st} percentile and at less than about the 81^{st} percentile of normalized PAI-1 levels for a randomized group of patients using any assay. In specific embodiments, a high level of PAI-1 corresponds to levels above a cut-off value of at least about the 65^{th}, 70^{th} or 75^{th} percentile. In an embodiment, the cut-off value for PAI-1 is at least about the 65^{th} percentile of normalized PAI-1 levels for a randomized group of cancer patients using any assay. In another embodiment, the cut-off value for PAI-1 is at least about the 70^{th} percentile of normalized PAI-1 levels for a randomized group of cancer patients using any assay. In another embodiment, the cut-off value for PAI-1 is at least about the 75^{th} percentile of normalized PAI-1 levels for a randomized group of cancer patients using any assay. A patient is classified as high risk if either or both of the uPA and PAI-1 levels are high. Conversely, a low level of uPA and a low level of PAI-1 correspond to levels that are lower than the cut-off value set for the indicator. A patient is classified as low risk if both the uPA and PAI-1 levels are low, *i.e*., below the cut-off value.

The antigen levels of uPA and PAI-1 in the analytes of primary tumor tissue extracts from a randomized group of patients are measured using an ELISA assay. High levels of uPA and/or PAI-1 can be defined as above cut-off levels set at a value for each of uPA and PAI-1 (not part of the claimed invention). Low levels of uPA and/or PAI-1 can be defined as below the cut-off value set for each of uPA and PAI-1. High level of uPA may be defined as above a cut-off level set at a value of at least about 2.4 ng/mg protein and not more than about 4 ng/uPA/mg protein. Specifically, a high level of uPA may be defined as above a cut-off value of at least about 2.6, 2.8, 3.0, 3.2, 3.2, 3.4, 3.6, or 3.8 ng uPA/mg protein. The cut-off value for uPA may be at least about 3 ng/uPA/mg protein, or at least about 3.5 ng/uPA/mg protein. High level of PAI-1 may be defined as above a cut-off level set at a value of at least about 11 ng/mg protein and not more than about 19 ng/PAI-1/mg protein. A high level of PAI-1 may be defined as above a cut-off value of at least about 13, 15, or 17 ng PAI-1/mg protein. Low level is defined as when both the uPA and PAI-1 levels are below the cut-off value. The number of patients that are above the set of cut-off values for uPA and PAI-1 corresponds to a percentage of patients that are above the set of cut-off values for uPA and PAI-1 in the randomized group of patients measured by the assay. High level of uPA is defined as above a cut-off level set at a value of at least about the 55^{th} percentile and not more than about the 75^{th} percentile for a randomized group of cancer patients using the assay. In specific embodiments, a high level of uPA corresponds to levels above a cut-off value of at least about the 60^{th}, 65^{th}, or 70^{th} percentile. High level of PAI-1 is defined as above a cut-off level set at a value of at least about the 61^{st} percentile and not more than about the 81^{st} percentile for a randomized group of patients using the assay. In specific embodiments, a high level of PAI-1 corresponds to levels above a cut-off value of at least about the 65^{th}, 70^{th}, or 75^{th} percentile. Low risk is defined as when both the uPA and PAI-1 levels are below the cut-off value. When different assays are used to measure the levels of uPA and PAI-1 in a randomized group of patients, the percentile cut-off values for uPA and PAI-1 corresponds to the percentage of patients that are above the set of cut-off values measured using the initial ELISA assay (or any number of assays, as long as the values are adjusted for differences in the assay) and the uPA and PAI-1 values obtained from a patient may be converted to percentile or even an analogous value for a different assay type using methods that are well known in the art to compare and normalize assay results. Hence, the cut-off levels can be measured as a percentile for a random group of patients where the levels of uPA and PAI-1 are measured using any assay.

A patient may be classified into high risk or low risk group depending on the level of uPA and level of PAI-1 as measured by the antigen levels of the analytes in a primary tumor tissue extracts of the patient using ELISA, particularly ELISA using American Diagnostica Inc. antibodies. A patient may also be classified into high risk or low risk group depending on the number of lymph nodes affected (nodal status). The number of lymph nodes affected may be 0, 1, 2, 3, 4, 5, 6, 7, 8, 9, 10, or more than 10.

Within a particular risk group, the patients may be further stratified by other clinically relevant criteria such as, but not limited to, menopausal status, tumor size, tumor grade, patient's age, hormone receptor status, other tumor biological factors, and any other factors that one skilled in the art considers in classifying cancer patients. These factors also contribute to the method of the present invention in selecting a treatment regimen for a node negative or node positive breast cancer patient. Such factors may also be used in identifying a comparable reference population for the predictive methods of the invention.

The method of the present invention relates to selecting from two or more treatment regimens, including a regimen of no treatment, a treatment regimen having the highest expected benefit for a node-positive patient with primary breast cancer. Optionally, the patient has undergone surgery to remove primary tumor tissue. The method comprises the steps of measuring the level of uPA and the level of PAI-1 or the levels of mRNA encoding uPA and PAI-1 in said patient or a tissue sample of said patient, including primary tumor tissue; classifying said patient as low risk or as high risk depending on the levels of uPA and PAI-1, *i.e.,* whether the levels are above or below a set cut-off value for each factor; and if said patient is classified as low risk, a treatment regimen is selected from the two or more treatment regimens that result in the highest expected benefit in a comparable population of low risk breast cancer patients; and, if said patient is classified as high risk, a treatment regimen is selected from said two or more treatment regimens that results in the highest expected benefit in a comparable population of high risk breast cancer patients.

The method of the present invention relates to selecting from two or more treatment regimens, including a regimen of no treatment, a treatment regimen having the highest demonstrated overall survival for a patient with cancer. Optionally, the patient has undergone surgery to remove primary tumor tissue. The method comprises measuring the level of uPA and the level of PAI-1 or the levels of mRNA encoding uPA and PAI-1 in said cancer patient or a tissue sample of said cancer patient; and classifying the patient as low or high risk depending upon whether the levels of uPA and PAI-1 or the levels of mRNA encoding uPA and PAI-1 are above or below a set cut-off level for each factor. If the patient is classified as low risk, then a treatment regimen is selected from said two or more treatment regimens that results in the highest demonstrated overall survival in a comparable population of low risk breast cancer patients; and if said patient is classified as high risk, then a treatment regimen is selected from said two or more treatment regimens that results in the highest demonstrated overall survival in a comparable population of high risk breast cancer patients.

The method of the present invention relates to selecting from two or more treatment regimens, including a regimen of no treatment, a treatment regimen having the highest demonstrated disease-free survival (preferably, long-term disease-free survival) for a patient. Optionally, the patient has undergone surgery to remove primary tumor tissue. The method comprises measuring the level of uPA and the level of PAI-1, preferably by ELISA, in said primary tumor tissue of said patient and classifying the patient as low or high risk; if said patient is classified as low risk, then a treatment regimen is selected from said two or more treatment regimens that results in the highest demonstrated disease-free survival in a comparable population of low risk breast cancer patients; and, if said patient is classified as high risk, then a treatment regimen is selected from said two or more treatment regimens that results in the highest demonstrated disease-free survival in a comparable population of high risk breast cancer patients. In a particular embodiment, said two or more treatment regimens do not include adjuvant CMF chemotherapy, or, in other embodiments, adjuvant chemotherapy.

The present invention also relates to methods for identifying subjects for a treatment regimen that benefits high risk patients. The method comprises the steps of measuring the level of uPA and the level of PAI-1 or the levels of mRNA encoding uPA and PAI-1 in one or more subjects or tissue samples of said subjects; and classifying the subjects as low or high risk; then one or more high risk subjects are selected for a treatment regimen, *i.e*., subjects in which the levels of both uPA and PAI-1 are above set cut-off values for uPA and PAI-1. The treatment regimen may include, but is not limited to, aggressive treatment regimens such as, adjuvant chemotherapy, adjuvant CMF chemotherapy, adjuvant non-CMF chemotherapy, adjuvant anthracyclin-containing chemotherapy, and adjuvant taxane-containing chemotherapy, and any other treatments that are associated with significant or debilitating or unpleasant side effects.

The present invention also relates to methods for identifying low risk subjects for a treatment regimen. The method comprises measuring the levels of uPA and PAI-1 or levels of mRNA encoding uPA and PAI-1 in a subject; classifying the patient as low or high risk; and selecting low risk subjects for the treatment regimen. The treatment regimens may include, but not limited to, non-aggressive treatment regimens such as, but are not limited to, non-treatment (except for initial surgery to remove tumor tissue), radiation therapy, adjuvant endocrine therapy such as tamoxifen therapy, immunotherapy and tumor-biological therapy, such as inhibitors/antagonists of uPA, and other treatments that have minor and/or tolerable side effects.

The applications also describes a method for predicting overall survival of a cancer patient undergoing a treatment regimen. Optionally, the treatment is provided after a primary tumor tissue has been removed. The method comprises measuring the level of uPA and the level of PAI-1 or the levels of mRNA encoding uPA and PAI-1 by any assay in the patient or a tissue sample of the patient; and classifying the patient as low or high risk. If the patient is classified as low risk, the overall survival for the patient is predicted as the average or mean or median overall survival of a comparable population of low risk patients having been administered said treatment regimen; and if the patient is classified as high risk, the overall survival for the patient is predicted as the average or mean or median overall survival of a comparable population of high risk patients having been administered said treatment regimen. In a preferred embodiment, the treatment regimen for a high risk patient is chemotherapy.

Also described is a method for predicting disease-free survival (preferably long-term disease-free survival) of a cancer patient undergoing a treatment regimen. Optionally, the treatment is provided after a primary tumor tissue has been removed. The method comprises measuring the level of uPA and the level of PAI-1 or the levels of mRNA encoding uPA and PAI-1 in said patient or a tissue sample of said patient, and classifying the patient as low or high risk. If the patient is classified as low risk, the disease-free survival for the patient is predicted as the average or mean or median disease-free survival of a comparable population of low risk patients having been administered said treatment regimen; and, if the patient is classified as high risk, the disease-free survival for said patient is predicted as the average or mean or median disease-free survival of a comparable population of high risk patients having been administered said treatment regimen. In a particular embodiment, the treatment regimen does not include adjuvant CMF chemotherapy. In a preferred embodiment, the treatment regimen for a high risk patient is chemotherapy.

The present invention also relates to a method for determining whether to administer an aggressive treatment to a cancer patient. Optionally, the treatment is provided after a primary tumor tissue has been removed. The method comprises measuring the level of uPA and the level of PAI-1 or the levels of mRNA encoding uPA and PAI-1, preferably by ELISA, in said primary tumor tissue of said patient, and classifying the patient as low or high risk. If the patient is classified as low risk, the aggressive treatment regimen is selected if it results in an expected benefit of treatment in a comparable population of low risk patients; and, if the patient is classified as high risk, an aggressive treatment regimen is selected that results in the highest expected benefit of treatment in a comparable population of high risk patients.

The methods of the present invention can be used to determine whether a subject can be administered an agent (*e.g*., an agonist, antagonist, peptidomimetic, protein, peptide, nucleic acid, small molecule, or other drug candidate) to treat cancer or other disease or disorder associated with high levels of uPA and PAI-1 for cancer patients. For example, such methods can be used to determine whether a subject can be effectively treated with a specific agent or class of agents (*e.g*., agents of a type which decrease activity or expression level of uPA and PAI-1 transcripts or polypeptides). In particular, treatment decision including therapy such as adjuvant systemic therapy for the high and low risk groups can be made when the treatment benefit is assessed for patients who receive the treatment regimen compared to those without such treatment regimen.

The present invention also relates to a method for predicting responses of a cancer patient to a treatment regimen. The method comprises measuring the level of uPA and the level of PAI-1 in said patient or a tissue sample of said patient, and classifying the patient as low or high risk. If the patient is classified as low risk, the benefit of the treatment for the patient is predicted to be the average or mean or median expected benefit in a comparable population of low risk patients having been administered said treatment regimen; and, if the patient is classified as high risk, the benefit of the treatment for the patient is predicted to be the average or mean or median expected benefit in a comparable population of high risk patients having been administered said treatment regimen. In a preferred embodiment, the treatment regimen for high risk patient is chemotherapy.

Furthermore, the levels of uPA and PAI-1 nucleic acid molecules or polypeptides can be correlated with the presence or expression level of other cancer-related proteins, such as for example, androgen receptor, estrogen receptor, adhesion molecules (*e.g*., E-cadherin), proliferation markers (*e.g*., MIB-1), tumor-suppressor genes (*e.g.,* TP53, retinoblastoma gene product), vascular endothelial growth factor (Lissoni et al., 2000, Int J Biol Markers. 15(4):308), Rad51 (Maacke et al., 2000, Int J Cancer. 88(6):907), cyclin D1, BRCA1, BRCA2, or carcinoembryonic antigen. These combined prognostic factors may further facilitate the classification of subjects into high and low risk subjects and using this information, to select a treatment regimen that is suitable for each group. In a preferred embodiment, chemotherapy is used in combination with other therapy such as hormonal therapy.

The methods described herein may be performed, for example, by utilizing pre-packaged diagnostic kits comprising at least one nucleic acid probe or antibody reagent described herein, which may be conveniently used, *e.g*., in clinical settings. Furthermore, any cell type or tissue, *e.g*., preferably cancerous breast cells or tissue, in which the cancer related gene is expressed may be utilized to measure the levels of uPA and PAI-1 gene products.

### 5.2. TYPES OF CANCERS

In various embodiments, the present invention provides methods for determining treatment regimens for breast cancer subjects.

In a preferred embodiment, the methods of the invention are directed at determining treatment regimen beyond surgical removal of tumor tissue for a breast cancer subject not having cancer cells detected in lymph node tissue.

The method comprises a step of measuring the uPA and PAI-1 levels in the tumor tissue in a representative collective group of patients with said malignancy. The cutoff levels for uPA and PAI-1 are then determined for the patients with the malignancy using methods well known to one skilled in the art, such as a log rank test. The patients are classified as high risk (*i.e*., patients in which the levels of either uPA or PAI-1, or both, are above set cut-off values for uPA and PAI-1) or low risk (*i.e*., patients in which the levels of both uPA and PAI are below set cut-off values for uPA and PAI-1). Then, a treatment regimen is selected from two or more treatments regimen for the low risk group that results in the highest expected benefit in a comparable population of low risk breast cancer patients. A treatment regimen is selected from two or more treatment regimens for the high risk group that results in the highest expected benefit in a comparable population of high risk breast cancer patients. The method further comprises considering other clinically relevant factors including nodal status, tumor size, tumor grade, patient's age, hormone receptor status, and menopausal status to select a treatment regimen of highest expected benefit.

The present invention provides a method of selecting a treatment regimen for a patient with breast cancer from two or more treatment regimens that provides the highest expected benefit to a patient with said malignant cancer, said method comprising the steps of measuring the level of uPA and PAI-1, preferably, by ELISA, in a primary tumor tissue of the patient and classifying the patient as low or high risk. If the patient is classified as low risk, a treatment regimen is selected if it results in the highest expected benefit in a comparable population of low risk patients with the malignancy; and if the patient is classified as high risk, a treatment regimen is selected if it results in the highest expected benefit in a comparable population of high risk patients with the malignancy.

### 5.3. DETECTING AND STAGING CANCER IN A SUBJECT

The methods of the present invention include measurement of the levels of naturally occurring uPA and PAI-1 polypeptides, or naturally occurring variants thereof, to classify breast cancer patients as high or low risk, so as to select a treatment regimen for breast cancer in a subject based upon predicted outcomes in comparable low or high risk populations. Comparable populations are identified using clinically relevant parameters such as the stage of breast cancer as discussed *supra.*

Staging refers to the grouping of patients according to the extent of their disease. Staging is useful in choosing treatment for individual patients, estimating prognosis, and comparing the results of different treatment programs. Staging of breast cancer for example is performed initially on a clinical basis, according to the physical examination and laboratory radiologic evaluation. The most widely used clinical staging system is the one adopted by the International Union against Cancer (UICC) and the American Joint Committee on Cancer (AJCC) Staging and End Results Reporting. It is based on the tumor-nodes-metastases (TNM) system as detailed in the 1988 *Manual for Staging of Cancer.* Breast cancer diseases or conditions which may be detected and/or staged in a subject according to the present invention include but are not limited to those listed in Table 2.

**TABLE 2**

| STAGING OF BREAST CANCER | | |
|---|---|---|
| T | PRIMARY TUMORS | |
| TX | Primary tumor cannot be assessed | |
| T0 | No evidence of primary tumor | |
| Tis | Carcinoma in situ: intraductal carcinoma, lobular carcinoma, or Paget's disease with no tumor | |
| T1 | Tumor 2 cm or less in its greatest dimension | |
| | a. | 0.5 cm or less in greatest dimension |
| | b. | Larger than 0.5 cm, but not larger than 1 cm in greatest dimension |
| | c. | Larger than 1 cm, but not larger than 2 cm in greatest dimension |
| T2 | Tumor more than 2 cm but not more than 5 cm in greatest dimension | |
| T3 | Tumor more than 5cm in its greatest dimension | |
| T4 | Tumor of any size with direct extension to chest wall or to skin. Chest wall includes ribs, intercostal muscles, and serratus anterior muscle, but not pectoral muscle. | |
| | a. | Extension to chest wall |
| | b. | Edema (including peau d'orange), ulceration of the skin of the breast, or satellite skin nodules confined to the same breast |
| | c. | Both of the above |
| | d. | Inflammatory carcinoma |
| Dimpling of the skin, nipple retraction, or any other skin changes except those in T4b may occur in T1, T2 or T3 without affecting the classification. | | |
| N | REGIONAL LYMPH NODES | |
| NX | Regional lymph nodes cannot be assessed (*e.g*., previously removed) | |
| N0 | No regional lymph node metastases | |
| N1 | Metastasis to movable ipsilateral axillary node(s) | |
| N2 | Metastases to ipsilateral axillary nodes fixed to one another or to other structures | |
| N3 | Metastases to ipsilateral internal mammary lymph node(s) | |
| M | DISTANT METASTASIS | |
| M0 | No evidence of distant metastasis | |
| M1 | Distant metastases (including metastases to ipsilateral supraclavicular lymph nodes) | |

One aspect of staging is assessing the nodal status. Specifically, patients are evaluated with respect to their nodal status being node negative or node positive. Node negative patients have no regional lymph node metastases. For node positive patients, the number of affected lymph nodes may be 1, 2, 3, 4, 5, 6, 7, 8, 9, 10 or more.

### 5.4. METHODS FOR TREATMENT OF CANCER

Described below are methods for treatment of cancer, *e.g*., breast cancer. The desired outcome of a treatment is at least to produce in a treated subject a healthful benefit, which in the case of cancer, including breast cancer, includes, but is not limited to, remission of the cancer, palliation of the symptoms of the cancer, and/or control of metastatic spread of the cancer, improvement in, or extension of the period of disease-free survival and/or overall survival.

### 5.4.1. CANCER TREATMENT REGIMENS

Cancer treatment regimens that may be used in the present invention include the use of one or more molecules, compounds or treatments for the treatment of cancer (*i.e*., cancer therapeutics), which molecules, compounds or treatments include, but are not limited to, surgery to remove tumor, chemoagents, immunotherapeutics, cancer vaccines, anti-angiogenic agents, cytokines, hormone therapies, gene therapies, blood cell transfusion, blood component transfusion and radiotherapies.

In one embodiment, one or more chemoagents are administered to treat a cancer patient. In a preferred embodiment, the chemoagent is not CMF. A chemoagent (or "anti-cancer agent" or "anti-tumor agent" or "cancer therapeutic") refers to any molecule or compound that assists in the treatment of tumors or cancer. Examples of chemoagents include, but are not limited to, bisphosphonate, cytosine arabinoside, taxoids (*e.g*., paclitaxel, docetaxel), anti-tubulin agents (*e.g*., paclitaxel, docetaxel, epothilone B, or its analogues), macrolides (*e.g*., rhizoxin) cisplatin, carboplatin, adriamycin, tenoposide, mitozantron, discodermolide, eleutherobine, 2-chlorodeoxyadenosine, alkylating agents (*e.g.,* cyclophosphamide, mechlorethamine, thioepa, chlorambucil, melphalan, carmustine (BSNU), lomustine (CCNU), cyclothosphamide, busulfan, dibromomannitol, streptozotocin, mitomycin C, and cis-dichlorodiamine platinum (II) (DDP) cisplatin, thio-tepa), antibiotics (*e.g*., dactinomycin (formerly actinomycin), bleomycin, mithramycin, anthramycin), antimetabolites (*e.g*., methotrexate, 6-mercaptopurine, 6-thioguanine, cytarabine, flavopiridol, 5-fluorouracil, fludarabine, gemcitabine, dacarbazine, temozolamide), asparaginase, diphtheria toxin, hexamethylmelamine, hydroxyurea, LYSODREN®, nucleoside analogues, plant alkaloids (*e.g*., Taxol, paclitaxel, camptothecin, topotecan, irinotecan (CAMPTOSAR, CPT-11), vincristine, vinca alkyloids such as vinblastine), podophyllotoxin (including derivatives such as epipodophyllotoxin, VP-16 (etoposide), VM-26 (teniposide)), cytochalasin B, colchine, gramicidin D, ethidium bromide, emetine, mitomycin, procarbazine, mechlorethamine, anthracyclines (*e.g*., daunorubicin (formerly daunomycin), doxorubicin, doxorubicin liposomal), dihydroxyanthracindione, mitoxantrone, mithramycin, actinomycin D, procaine, tetracaine, lidocaine, propranolol, puromycin, anti-mitotic agents, abrin, ricin A, pseudomonas exotoxin, nerve growth factor, platelet derived growth factor, tissue plasminogen activator, aldesleukin, allutamine, anastrozle, bicalutamide, biaomycin, busulfan, capecitabine, carboplatin, chlorabusil, cladribine, cylarabine, daclinomycin, estramusine, floxuridhe, gamcitabine, gosereine, idarubicin, itosfamide, lauprolide acetate, levamisole, lomusline, mechlorethamine, magestrol, acetate, mercaptopurino, mesna, mitolanc, pegaspergase, pentoslatin, picamycin, riuxlmab, campath-1, straplozocin, thioguanine, tretinoin, vinorelbine, or any fragments, family members, or derivatives thereof, including pharmaceutically acceptable salts thereof. Compositions comprising a combination of chemoagents (*e.g*., AC, EC, FEC, ET, E-Docetaxel, Docetaxel-Xeloda etc.) may also be used to treat cancer. AC and EC comprise adriamycin or epirubicin and cyclophosphamide; FAC and FEC comprise fluoruracil, adriamycin or epirubicin, and cyclophosphamide; and ET comprises epirubicin and taxol.

Individual cytotoxic or cytostatic agents that may be used to treat cancer include but are not limited to an androgen, asparaginase, 5-azacytidine, azathioprine, buthionine sulfoximine, CC-1065, chlorambucil, colchicine, an estrogen, 5-fluordeoxyuridine, nitroimidazole, and thioTEPA.

In other embodiments, the treatment regimens for breast cancer include pharmaceutical compositions comprising 5-fluorouracil, cisplatin, docetaxel, doxorubicin, Herceptin®, gemcitabine (Seidman, 2001, Oncology 15:11-14), IL-2, paclitaxel, and/or VP-16 (etoposide).

In a preferred embodiment, the treatment regimen comprises chemotherapy using any of the above listed chemoagent in combination with other treatment regimens for breast cancer listed above. In another preferred embodiment, the treatment regimen comprises chemotherapy in combination with HERCEPTIN® and/or drugs or small molecules that target uPA and/or PAI-1 as discussed *infra.*

In one embodiment, the chemoagent used is gemcitabine at a dose ranging from 100 to 1000 mg/m²/cycle. In one embodiment, the chemoagent used is dacarbazine at a dose ranging from 200 to 4000 mg/m²/cycle. In a preferred embodiment, the dose ranges from 700 to 1000 mg/m²/cycle. In another embodiment, the chemoagent used is fludarabine at a dose ranging from 25 to 50 mg/m²/cycle. In another embodiment, the chemoagent used is cytosine arabinoside (Ara-C) at a dose ranging from 200 to 2000 mg/m²/cycle. In another embodiment, the chemoagent used is docetaxel at a dose ranging from 1.5 to 7.5 mg/kg/cycle. In another embodiment, the chemoagent used is paclitaxel at a dose ranging from 5 to 15 mg/kg/cycle. In yet another embodiment, the chemoagent used is cisplatin at a dose ranging from 5 to 20 mg/kg/cycle. In yet another embodiment, the chemoagent used is 5-fluorouracil at a dose ranging from 5 to 20 mg/kg/cycle. In yet another embodiment, the chemoagent used is doxorubicin at a dose ranging from 2 to 8 mg/kg/cycle. In yet another embodiment, the chemoagent used is epipodophyllotoxin at a dose ranging from 40 to 160 mg/kg/cycle. In yet another embodiment, the chemoagent used is cyclophosphamide at a dose ranging from 50 to 200 mg/kg/cycle. In yet another embodiment, the chemoagent used is irinotecan at a dose ranging from 50 to 75, 75 to 100, 100 to 125, or 125 to 150 mg/m²/cycle. In yet another embodiment, the chemoagent used is vinblastine at a dose ranging from 3.7 to 5.4, 5.5 to 7.4, 7.5 to 11, or 11 to 18.5 mg/m²/cycle. In yet another embodiment, the chemoagent used is vincristine at a dose ranging from 0.7 to 1.4, or 1.5 to 2 mg/m²/cycle. In yet another embodiment, the chemoagent used is methotrexate at a dose ranging from 3.3 to 5, 5 to 10, 10 to 100, or 100 to 1000 mg/m²/cycle. The dosages for breast cancer may be found in any standard practitioner's handbook (e.g., Breast Disease (J. Harris, editor) and the current guidelines provided by St. Gallen or the National Institute of Health.

In a preferred embodiment, the invention further encompasses the use of low doses of chemoagents treatment regimen. For example, a low dose (*e.g*., 6 to 60 mg/m²/day or less) of docetaxel is administered to a cancer patient. In another embodiment, a low dose (*e.g*., 10 to 135 mg/m²/day or less) of paclitaxel is administered to a cancer patient. In yet another embodiment, a low dose (*e.g*., 2.5 to 25 mg/m²/day or less) of fludarabine is administered to a cancer patient. In yet another embodiment, a low dose (*e.g*., 0.5 to 1.5 g/m²/day or less) of cytosine arabinoside (Ara-C) is administered to a cancer patient.

In one embodiment, the chemoagent used is cisplatin, *e.g*., PLATINOL™ or PLATINOL-AQ™ (Bristol Myers), at a dose ranging from 5 to 10, 10 to 20, 20 to 40, or 40 to 75 mg/m²/cycle. In another embodiment, a dose of cisplatin ranging from 7.5 to 75 mg/m²/cycle is administered to a patient with ovarian cancer or other cancer. In another embodiment, a dose of cisplatin ranging from 5 to 50 mg/m²/cycle is administered to a patient with bladder cancer or other cancer.

In another embodiment, the chemoagent used is carboplatin, *e.g*., PARAPLATIN™ (Bristol Myers), at a dose ranging from 2 to 4, 4 to 8, 8 to 16, 16 to 35, or 35 to 75 mg/m²/cycle. In another embodiment, a dose of carboplatin ranging from 7.5 to 75 mg/m²/cycle is administered to a patient with ovarian cancer or other cancer. In another embodiment, a dose of carboplatin ranging from 5 to 50 mg/m²/cycle is administered to a patient with bladder cancer or other cancer. In another embodiment, a dose of carboplatin ranging from 2 to 20 mg/m²/cycle is administered to a patient with testicular cancer or other cnacer.

In another embodiment, the chemoagent used is docetaxel, *e.g*., TAXOTERE™ (Rhone Poulenc Rorer) at a dose ranging from 6 to 10, 10 to 30, or 30 to 60 mg/m²/cycle.

In another embodiment, the chemoagent used is paclitaxel, *e.g*., TAXOL™ (Bristol Myers Squibb), at a dose ranging from 10 to 20, 20 to 40, 40 to 70, or 70 to 135 mg/kg/cycle.

In another embodiment, the chemoagent used is 5-fluorouracil at a dose ranging from 0.5 to 5 mg/kg/cycle.

In another embodiment, the chemoagent used is doxorubicin, *e.g*., ADRIAMYCIN™ (Pharmacia & Upjohn), DOXIL (Alza), RUBEX™ (Bristol Myers Squibb), at a dose ranging from 2 to 4, 4 to 8, 8 to 15, 15 to 30, or 30 to 60 mg/kg/cycle.

In another embodiment, the treatment regimen includes one or more immunotherapeutic agents, such as antibodies and immunomodulators, which include, but are not limited to, HERCEPTIN® , RITUXAN®, OVAREX™, PANOREX®, BEC2, IMC-C225, VITAXIN™, CAMPATH® I/H, Smart MI95, LYMPHOCIDE™, Smart I D10, and ONCOLYM™, rituximab, gemtuzumab, or trastuzumab.

In another embodiment, the treatment regimen includes one or more anti-angiogenic agents, which include, but are not limited to, angiostatin, thalidomide, kringle 5, endostatin, Serpin (Serine Protease Inhibitor) anti-thrombin, 29 kDa N-terminal and a 40 kDa C-terminal proteolytic fragments of fibronectin, 16 kDa proteolytic fragment of prolactin, 7.8 kDa proteolytic fragment of platelet factor-4, a 13-amino acid peptide corresponding to a fragment of platelet factor-4 (Maione et al., 1990, Cancer Res. 51:2077), a 14-amino acid peptide corresponding to a fragment of collagen I (Tolma et al., 1993, J. Cell Biol. 122:497), a 19 amino acid peptide corresponding to a fragment of Thrombospondin I (Tolsma et al., 1993, J. Cell Biol. 122:497), a 20-amino acid peptide corresponding to a fragment of SPARC (Sage et al., 1995, J. Cell. Biochem. 57:1329-), or any fragments, family members, or derivatives thereof, including pharmaceutically acceptable salts thereof.

Other peptides that inhibit angiogenesis and correspond to fragments of laminin, fibronectin, procollagen, and EGF have also been described (*see* the review by Cao, 1998, Prog. Mol. Subcell. Biol. 20:161). Monoclonal antibodies and cyclic pentapeptides, for example, VITAXIN™, which block certain integrins that bind RGD proteins (*i.e*., possess the peptide motif Arg-Gly-Asp), have been demonstrated to have anti-vascularization activities (Brooks et al., 1994, Science 264:569; Hammes et al., 1996, Nature Medicine 2:529). Moreover, inhibition of the urokinase plasminogen activator receptor by receptor antagonists inhibits angiogenesis, tumor growth and metastasis (Min et al., 1996, Cancer Res. 56:2428-33; Crowley et al., 1993, Proc Natl Acad Sci. USA 90:5021). Use of such anti-angiogenic agents is also contemplated by the present invention.

In another embodiment, the treatment regimen includes radiation.

In another embodiment, the treatment regimen includes administration of one or more cytokines, which includes, but is not limited to, lymphokines, tumor necrosis factors, tumor necrosis factor-like cytokines, lymphotoxin-a, lymphotoxin-b, interferon-a, interferon-b, macrophage inflammatory proteins, granulocyte monocyte colony stimulating factor, interleukins (including, but not limited to, interleukin-1, interleukin-2, interleukin-6, interleukin-12, interleukin-15, interleukin-18), OX40, CD27, CD30, CD40 or CD137 ligands, Fas-Fas ligand, 4-1BBL, endothelial monocyte activating protein or any fragments, family members, or derivatives thereof, including pharmaceutically acceptable salts thereof.

In yet another embodiment, the treatment regimen includes hormonal treatment. Hormonal therapeutic treatments comprise hormonal agonists, hormonal antagonists (*e.g*., flutamide, tamoxifen, leuprolide acetate (LUPRON™), LH-RH antagonists), inhibitors of hormone biosynthesis and processing, steroids (*e.g*., dexamethasone, retinoids, betamethasone, cortisol, cortisone, prednisone, dehydrotestosterone, glucocorticoids, mineralocorticoids, estrogen, testosterone, progestins), antigestagens (*e.g*., mifepristone, onapristone), and antiandrogens (*e.g*., cyproterone acetate).

In one embodiment, the treatment regimen includes administration of at least one cancer therapeutic agent, for a short treatment cycle to a cancer patient to treat cancer. The duration of treatment with the cancer therapeutic agent may vary according to the particular cancer therapeutic agent used. The invention also contemplates discontinuous administration or daily doses divided into several partial administrations. An appropriate treatment time for a particular cancer therapeutic agent will be appreciated by the skilled artisan, and the invention contemplates the continued assessment of optimal treatment schedules for each cancer therapeutic agent.

The present invention contemplates at least one cycle, preferably more than one cycle during which the treatment regimen is carried out. An appropriate period of time for one cycle will be appreciated by the skilled artisan, as will the total number of cycles, and the interval between cycles. The invention contemplates the continued assessment of optimal treatment regimen and cancer therapeutic agent.

### 5.4.2. GENE THERAPY

Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIBTECH 11(5):155-215). Methods commonly known in the art of recombinant DNA technology which can be used are described in Ausubel et al. (eds.), 1993, Current Protocols in Molecular Biology, John Wiley & Sons, NY; Kriegler, 1990, Gene Transfer and Expression, A Laboratory Manual, Stockton Press, NY; and in Chapters 12 and 13, Dracopoli et al. (eds), 1994, Current Protocols in Human Genetics, John Wiley & Sons, NY.

Antisense and ribozyme molecules which inhibit oncogene expression or genes that are upregulated in cancer cells can be used as therapeutic nucleic acids in accordance with the invention for the treatment of cancer. Techniques for the production and use of such molecules are well known to those of skill in the art.

In a specific embodiment, nucleic acids comprising a sequence encoding an antisense or ribozyme molecule which inhibit the expression of oncogenes or genes that are upregulated in cancer cells are administered to treat cancer.

In one aspect, the therapeutic nucleic acid comprises an expression vector that expresses the antisense or ribozyme molecule (or fragment thereof) in a suitable host. In particular, such a nucleic acid comprises a promoter, said promoter being inducible or constituitive, and, optionally, tissue-specific.

Delivery of the nucleic acid into a patient may be either direct, in which case the patient is directly exposed to the nucleic acid or nucleic acid-carrying vector or a delivery complex, or indirect, in which case, cells are first transformed with the nucleic acid *in vitro*, then transplanted into the patient. These two approaches are known, respectively, as *in vivo* or *ex vivo* gene therapy.

In a specific embodiment, the nucleic acid is directly administered *in vivo*, where it is expressed to produce the antisense or ribozyme molecules. This can be accomplished by any of numerous methods known in the art, *e.g*., by constructing it as part of an appropriate nucleic acid expression vector and administering it so that it becomes intracellular, *e.g*., by infection using a defective or attenuated retroviral or other viral vector (see U.S. Patent No. 4,980,286), or by direct injection of naked DNA, or by use of microparticle bombardment (*e.g*., a gene gun; Biolistic, Dupont), or coating with lipids or cell-surface receptors or transfecting agents, encapsulation in biopolymers (*e.g*., poly-β-1->4-N-acetylglucosamine polysaccharide; see U.S. Patent No. 5,635,493), encapsulation in liposomes, microparticles, or microcapsules, or by administering it in linkage to a peptide which is known to enter the nucleus, by administering it in linkage to a ligand which is known to enter the nucleus, by administering it in linkage to a ligand subject to receptor-mediated endocytosis (see *e.g*., Wu and Wu, 1987, J. Biol. Chem. 262:4429-4432), etc. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g*., PCT Publications WO 92/06180 dated April 16, 1992 (Wu et al.); WO 92/22635 dated December 23, 1992 (Wilson et al.); WO92/20316 dated November 26, 1992 (Findeis et al.); WO93/14188 dated July 22, 1993 (Young). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

Adenoviruses are other viral vectors that can be used in gene therapy. Adenoviruses are especially attractive vehicles for delivering genes to respiratory epithelia where they cause a mild disease. Other targets for adenovirus-based delivery systems are liver, the central nervous system, endothelial cells, and muscle. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252-431-434; Rosenfeld et al., 1992, Cell 68:143-155; and Mastrangeli et al., 1993, J. Clin. Invest. 91:225-234. Adeno-associated virus (AAV) has also been proposed for use in gene therapy (Walsh et al., 1993, Proc. Soc. Exp. Biol. Med. 204:289-300).

Antisense approaches involve the design of oligonucleotides (either DNA or RNA) that are complementary to a target mRNA, e.g., oncogenes, genes that are upregulated in cancer cells, such as uPA or PAI-1. The antisense oligonucleotides will bind to the complementary oncogene mRNA transcripts and prevent translation. Absolute complementarity, although preferred, is not required. A sequence "complementary" to a portion of an RNA, as referred to herein, means a sequence having sufficient complementarity to be able to hybridize with the non-poly A portion of the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the message, *e.g*., the 5' untranslated sequence up to and including the AUG initiation codon, should work most efficiently at inhibiting translation. However, sequences complementary to the 3' untranslated sequences of mRNAs have also been shown to be effective at inhibiting translation ofmRNAs as well. *(See generally,* Wagner, R., 1994, Nature 372:333).

Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Antisense nucleic acids should be at least six nucleotides in length, and are preferably oligonucleotides ranging from 6 to about 50 nucleotides in length. In specific aspects, the oligonucleotide is at least 10 nucleotides, at least 17 nucleotides, at least 25 nucleotides or at least 50 nucleotides.

Regardless of the choice of target sequence, it is preferred that *in vitro* studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

Oligonucleotides that may be used in connection with the treatment method may be synthesized by standard methods known in the art, *e.g*., by use of an automated DNA synthesizer (such as are commercially available from Biosearch, Applied Biosystems, *etc*.). As examples, phosphorothioate oligonucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. U.S.A. 85:7448), *etc*.

However, it is often difficult to achieve intracellular concentrations of the antisense sufficient to suppress translation of endogenous mRNAs. Therefore a preferred approach utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the target gene transcripts and thereby prevent translation of the target gene mRNA. For example, a vector can be introduced *in vivo* such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, 1981, Nature 290:304), the promoter contained in the 3' long terminal repeat of Rous sarcoma virus (Yamamoto et al., 1980, Cell 22:787), the herpes thymidine kinase promoter (Wagner et al., 1981, Proc. Natl. Acad. Sci. USA 78:1441), the regulatory sequences of the metallothionein gene (Brinster et al., 1982, Nature 296:39), etc. Any type of plasmid, cosmid, YAC or viral vector can be used to prepare the recombinant DNA construct which can be introduced directly into the tissue site. Alternatively, viral vectors can be used which selectively infect the desired tissue.

The effective dose of target antisense oligonucleotide to be administered during a treatment cycle ranges from about 0.01 to 0.1, 0.1 to 1, or 1 to 10 mg/kg/day. The dose of target antisense oligonucleotide to be administered can be dependent on the mode of administration. For example, intravenous administration of a target antisense oligonucleotide would likely result in a significantly higher full body dose than a full body dose resulting from a local implant containing a pharmaceutical composition comprising the target antisense oligonucleotide. In one embodiment, the target antisense oligonucleotide is administered subcutaneously at a dose of 0.01 to 10 mg/kg/day. In another embodiment, the target antisense oligonucleotide is administered intravenously at a dose of 0.01 to 10 mg/kg/day. In yet another embodiment, the target antisense oligonucleotide is administered locally at a dose of 0.01 to 10 mg/kg/day. It will be evident to one skilled in the art that local administrations can result in lower total body doses. For example; local administration methods such as intratumor administration, intraocular injection, or implantation, can produce locally high concentrations of target antisense oligonucleotide, but represent a relatively low dose with respect to total body weight. Thus, in such cases, local administration of the target antisense oligonucleotide is contemplated to result in a total body dose of about 0.01 to 5 mg/kg/day.

In another embodiment, a particularly high dose of target antisense oligonucleotide, which ranges from about 10 to 50 mg/kg/day, is administered during a treatment cycle.

Moreover, the effective dose of a particular target antisense oligonucleotide may depend on additional factors, including the type of cancer, the stage of the cancer, the oligonucleotide's toxicity, the oligonucleotide's rate of uptake by cancer cells, as well as the weight, age, and health of the individual to whom the antisense oligonucleotide is to be administered. Because of the many factors present *in vivo* that may interfere with the action or biological activity of the target antisense oligonucleotide, one of ordinary skill in the art can appreciate that an effective amount of the target antisense oligonucleotide may vary for each individual.

A "low dose" or "reduced dose" refers to a dose that is below the normally administered range, *i.e*., below the standard dose as suggested by the Physicians' Desk Reference, 54th Edition (2000) or a similar reference. Such a dose can be sufficient to inhibit cell proliferation, or demonstrates ameliorative effects in a human, or demonstrates efficacy with fewer side effects as compared to standard cancer treatments. Normal dose ranges used for particular therapeutic agents and standard cancer treatments employed for specific diseases can be found in the Physicians' Desk Reference, 54th Edition (2000) or in Cancer: Principles & Practice of Oncology, DeVita, Jr., Hellman, and Rosenberg (eds.) 2nd edition, Philadelphia, PA: J.B. Lippincott Co., 1985.

A "treatment cycle" or "cycle" refers to a period during which a single therapeutic or sequence of therapeutics is administered. In some instances, one treatment cycle may be desired, such as, for example, in the case where a significant therapeutic effect is obtained after one treatment cycle. The present invention contemplates at least one treatment cycle, generally preferably more than one treatment cycle.

Other factors to be considered in determining an effective dose of target antisense oligonucleotide include whether the oligonucleotide will be administered in combination with other therapeutics. In such cases, the relative toxicity of the other therapeutics may indicate the use of target antisense oligonucleotide at low doses. Alternatively, treatment with a high dose of target antisense oligonucleotide can result in combination therapies with reduced doses of therapeutics. In a specific embodiment, treatment with a particularly high dose of target antisense oligonucleotide can result in combination therapies with greatly reduced doses of cancer therapeutics. For example, treatment of a patient with 10, 20, 30, 40, or 50 mg/kg/day of target antisense oligonucleotide can further increase the sensitivity of a subject to cancer therapeutics. In such cases, the particularly high dose of target antisense oligonucleotide is combined with, for example, a greatly shortened radiation therapy schedule. In another example, the particularly high dose of target antisense oligonucleotide produces significant enhancement of the potency of cancer therapeutic agents.

In one embodiment, gene therapy with recombinant cells secreting interleukin-2 is administered to prevent or treat cancer, particularly breast cancer (*See,* e.g. Deshmukh et al., 2001, J. Neurosurg. 94:287).

The invention contemplates other treatment regimens depending on the particular target antisense oligonucleotide to be used, or depending on the particular mode of administration, or depending on whether the target antisense oligonucleotide is administered as part of a combination therapy, *e.g*., in combination with a cancer therapeutic agent. The daily dose can be administered in one or more treatments.

Ribozyme molecules which are complementary to RNA sequences coded for by a target gene such as oncogenes or genes that are upregulated in cancer cells can be used to treat any cancer, including breast cancer.

Ribozymes are enzymatic RNA molecules capable of catalyzing the specific cleavage of RNA (For a review see, for example Rossi, J., 1994, Current Biology 4:469). The mechanism of ribozyme action involves sequence specific or selective hybridization of the ribozyme molecule to complementary target RNA, followed by a endonucleolytic cleavage. The composition of ribozyme molecules must include one or more sequences complementary to the target gene mRNA, and must include the well known catalytic sequence responsible for mRNA cleavage (*See* U.S. Pat. No. 5,093,246). As such, useful ribozyme molecules may be engineered hammerhead motif ribozyme molecules that specifically and efficiently catalyze endonucleolytic cleavage of RNA sequences encoding target gene proteins. Ribozyme molecules designed to catalytically cleave the target mRNA transcripts can also be used to prevent translation of target mRNA and expression of target or pathway gene. (*See, e.g.,* PCT International Publication WO90/11364, published October 4, 1990; Sarver et al., 1990, Science 247:1222). While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, 1988, Nature 334:585. Preferably the ribozyme is engineered so that the cleavage recognition site is located near the 5' end of the target mRNA; *i.e*., to increase efficiency and minimize the intracellular accumulation of non-functional mRNA transcripts.

As in the antisense approach, the ribozymes can be composed of modified oligonucleotides (*e.g*., for improved stability, targeting, etc.) and should be delivered to cells which express the target gene *in vivo.* A preferred method of delivery involves using a DNA construct "encoding" the ribozyme under the control of a strong constitutive pol III or pol II promoter, so that transfected cells will produce sufficient quantities of the ribozyme to destroy endogenous target gene messages and inhibit translation. Because ribozymes unlike antisense molecules, are catalytic, a lower intracellular concentration is required for efficiency.

Anti-sense RNA and DNA, ribozyme, can be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules can be generated by *in vitro* and *in vivo* transcription of DNA sequences encoding the antisense RNA molecule. Such DNA sequences can be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

### 5.4.3. THERAPEUTIC ANTIBODIES

Antibodies that binds specifically to target proteins such as oncogenes, genes that are uprcgulated in cancer cells, including, for example, uPA and PAI-1, or cancer antigens, can be utilized to treat breast cancer. Such antibodies can be generated using standard techniques described in Section 5.7.2, *infra*, against full length wild type or mutant target proteins, or against peptides corresponding to portions of the proteins. The antibodies include but are not limited to polyclonal, monoclonal, Fab fragments, single chain antibodies, chimeric antibodies, and the like.

Antibodies that recognize any epitope on the target protein can be used as therapy against cancer.

For target proteins that are expressed as an intracellular proteins, it is preferred that internalizing antibodies be used. However, lipofectin or liposomes can be used to deliver the antibody or a fragment of the Fab region which binds to the target epitope into cells. Where fragments of the antibody are used, the smallest inhibitory fragment which binds to the target protein is preferred. For example, peptides having an amino acid sequence corresponding to the domain of the variable region of the antibody that binds to a target protein can be used. Such peptides can be synthesized chemically or produced via recombinant DNA technology using methods well known in the art (*e.g., see* Creighton, 1983, *supra;* and Sambrook *et al.,* 1989, *supra*). Alternatively, single chain antibodies, such as neutralizing antibodies, which bind to intracellular epitopes can also be administered. Such single chain antibodies can be administered, for example, by expressing nucleotide sequences encoding single-chain antibodies within the target cell population by utilizing, for example, techniques such as those described in Marasco *et al.* (Marasco,. et al., 1993, Proc. Natl. Acad. Sci. USA 90:7889).

For example, but not by way of limitation, cancers and tumors associated with the following cancer and tumor antigens may be treated by administration of therapeutic antibodies that recognizes these cancer antigens: KS 1/4 pan-carcinoma antigen (Perez and Walker, 1990, J. Immunol. 142:3662-3667; Bumal, 1988, Hybridoma 7(4):407-415), ovarian carcinoma antigen (CA125) (Yu et al., 1991, Cancer Res. 51(2):468-475), prostatic acid phosphate (Tailor et al., 1990, Nucl. Acids Res. 18(16):4928*),* prostate specific antigen (Henttu and Vihko, 1989, Biochem. Biophys. Res. Comm. 160(2):903-910; Israeli et al, 1993, Cancer Res. 53:227-230), melanoma-associated antigen p97 (Estin et al., 1989, J. Natl. Cancer Instit. 81(6):445-446), melanoma antigen gp75 (Vijayasardahl et al., 1990, J. Exp. Med 171 (4):1375-1380), high molecular weight melanoma antigen (HMW-MAA) (Natali et al., 1987, Cancer 59:55-63; Mittelman et al., 1990, J. Clin. Invest. 86:2136-2144), prostate specific membrane antigen, carcinoembryonic antigen (CEA) (Foon et al., 1994, Proc. Am. Soc. Clin. Oncol. 13:294), polymorphic epithelial mucin antigen, human milk fat globule antigen, colorectal tumor-associated antigens such as: CEA, TAG-72 (Yokata et al., 1992, Cancer Res. 52:3402-3408), CO17-1A (Ragnhammar et al., 1993, Int. J. Cancer 53:751-758); GICA 19-9 (Herlyn et al., 1982, J. Clin. Immunol. 2:135), CTA-1 and LEA, Burkitt's lymphoma antigen-38.13, CD19 (Ghetie et al., 1994, Blood 83:1329-1336), human B-lymphoma antigen-CD20 (Reffet al., 1994, Blood 83:435-445), CD33 (Sgouros et al., 1993, J. Nucl. Med. 34:422-430), melanoma specific antigens such as ganglioside GD2 (Saleh et al., 1993, J.Immunol., 151, 3390-3398), ganglioside GD3 (Shitara et al., 1993, Cancer Immunol. Immunother. 36:373-380), ganglioside GM2 (Livingston et al., 1994, J. Clin. Oncol. 12:1036-1044), ganglioside GM3 (Hoon et al., 1993, Cancer Res. 53:5244-5250), tumor-specific transplantation type of cell-surface antigen (TSTA) such as virally-induced tumor antigens including T-antigen DNA tumor viruses and Envelope antigens of RNA tumor viruses, oncofetal antigen-alpha-fetoprotein such as CEA of colon, bladder tumor oncofetal antigen (Hellstrom et al., 1985, Cancer. Res. 45:2210-2188), differentiation antigen such as human lung carcinoma antigen L6, L20 (Hellstrom et al., 1986, Cancer Res. 46:3917-3923), antigens of fibrosarcoma, human leukemia T cell antigen-Gp37 (Bhattacharya-Chatterjee et al., 1988, J. of Immunospecifically. 141:1398-1403), neoglycoprotein, sphingolipids, breast cancer antigen such as EGFR (Epidermal growth factor receptor), HER2 antigen (p185^{HERZ}), polymorphic epithelial mucin (PEM) (Hilkens et al., 1992, Trends in Bio. Chem. Sci. 17:359), malignant human lymphocyte antigen-APO-1 (Bernhard et al., 1989, Science 245:301-304), differentiation antigen (Feizi, 1985, Nature 314:53-57) such as I antigen found in fetal erythrocytes, primary endoderm, I antigen found in adult erythrocytes, preimplantation embryos, I(Ma) found in gastric adenocarcinomas, M18, M39 found in breast epithelium, SSEA-1 found in myeloid cells, VEP8, VEP9, Myl, VIM-D5, D₁56-22 found in colorectal cancer, TRA-1-85 (blood group H), C14 found in colonic adenocarcinoma, F3 found in lung adenocarcinoma, AH6 found in gastric cancer, Y hapten, Le^{y} found in embryonal carcinoma cells, TL5 (blood group A), EGF receptor found in A431 cells, E₁ series (blood group B) found in pancreatic cancer, FC10.2 found in embryonal carcinoma cells, gastric adenocarcinoma antigen, CO-514 (blood group Le^{a}) found in Adenocarcinoma, NS-10 found in adenocarcinomas, CO-43 (blood group Le^{b}), G49 found in EGF receptor of A431 cells, MH2 (blood group ALe^{b}/Le^{y}) found in colonic adenocarcinoma, 19.9 found in colon cancer, gastric cancer mucins, T₅A₇ found in myeloid cells, R₂₄ found in melanoma, 4.2, G_{D3}, D1.1, OFA-1, G_{M2}, OFA-2, G_{D2}, and M1:22:25:8 found in embryonal carcinoma cells, and SSEA-3 and SSEA-4 found in 4 to 8-cell stage embryos. In one embodiment, the antigen is a T-cell receptor derived peptide from a Cutaneous Tcell Lymphoma (see, Edelson, 1998, The Cancer Journal 4:62).

The subject being treated one cancer treatment may, optionally, be treated with other cancer treatments such as radiation therapy or chemotherapy. In particular, the treatment regimen that may be used in the present invention may be administered in conjunction with one or a combination of chemotherapeutic agents as described above.

Antibodies that are conjugated to a cytostatic and/or a cytotoxic agent may be used in the treatment of cancer. A useful class of cytotoxic or cytostatic agents for practicing the therapeutic regimens of the present invention, by conjugation to an antibody, include, but are not limited to, the following non-mutually exclusive classes of agents: alkylating agents, anthracyclines, antibiotics, antifolates, antimetabolites, antitubulin agents, auristatins, chemotherapy sensitizers, DNA minor groove binders, DNA replication inhibitors, duocarmycins, etoposides, fluorinated pyrimidines, lexitropsins, nitrosoureas, platinols, purine antimetabolites, puromycins, radiation sensitizers, steroids, ricin toxin, radionuclide, taxanes, topoisomerase inhibitors, and vinca alkaloids or any other agent effective to kill and arrest cancer or tumor cell growth.

In a preferred cancer treatment, the cytotoxic or cytostatic agent that is attached to a therapeutic antibody is an antimetabolite. The antimetabolite can be a purine antagonist (*e.g*., azothioprine) or mycophenolate mofetil), a dihydrofolate reductase inhibitor (*e.g*., methotrexate), acyclovir, gangcyclovir, zidovudine, vidarabine, ribavarin, azidothymidine, cytidine arabinoside, amantadine, dideoxyuridine, iododeoxyuridine, poscamet, and trifluridine.

Techniques for conjugating such therapeutic moieties to proteins, and in particular to antibodies, are well known, *see, e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc., 1985); Hellstrom et al., "Antibodies For Drug Delivery", in Controlled Drug Delivery (2nd ed.), Robinson et al. (eds.), pp. 623-53 (Marcel Dekker, Inc., 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., 1982, Immunol. Rev. 62:119-58.

### 5.4.4. VACCINE THERAPY

The treatment regimen may also include administration of vaccines to a subject that effectively stimulates an immune response against cancer antigens such as those listed in Section 5.4.3. The invention thus contemplates the use of treatment regimen of vaccinating a subject against cancer wherein said subject is at risk of a recurrence of breast cancer.

Many methods may be used to introduce the vaccine formulations, these include but are not limited to intranasal, intratracheal, oral, intradermal, intramuscular, intraperitoneal, intravenous, and subcutaneous route. Various adjuvants may be used to increase the immunological response, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

In one embodiment, the treatment regimen for metastatic carcinoma includes *ex vivo* gene therapy or "cancer vaccine". Cancer cells are isolated from patients, transduced with various gene vectors and expanded *in vitro.* After irradiation, the cells are transplanted autologously to enhance the patient's immune response against the tumor.

Another treatment regimen that may be used includes genetic immunization. Genetic immunization is particularly advantageous as it stimulates a cytotoxic T-cell response but does not utilize live attenuated vaccines, which can revert to a virulent form and infect the host causing complications from infection. As used herein, genetic immunization comprises inserting the nucleotides of a target gene, such as an oncogene, or any antigen listed in Section 5.4.3, into a host, such that the nucleotides are taken up by cells of the host and the proteins encoded by the nucleotides are translated. These translated proteins are then either secreted or processed by the host cell for presentation to immune cells and an immune reaction is stimulated. Preferably, the immune reaction is a cytotoxic T cell response, however, a humoral response or macrophage stimulation is also useful in preventing initial or additional tumor growth and metastasis or spread of the cancer. The skilled artisan will appreciate that there are various methods for introducing foreign nucleotides into a host animal and subsequently into cells for genetic immunization, for example, by intramuscular injection of about 50 mg of plasmid DNA encoding the proteins of an oncogene solubilized in 50 ml of sterile saline solution, with a suitable adjuvant (*See, e.g.,* Weiner and Kennedy, 1999, Scientific American 7:50-57; Lowrie et al., 1999, Nature 400:269-271).

The treatment regimen that may be used includes a vaccine formulation comprising an immunogenic amount of an oncogene product.

### 5.5. EFFECTIVE DOSE

Toxicity and therapeutic efficacy of compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects can be used, care should be taken to design a delivery system that targets such compounds to the site of affected tissue in order to minimize potential damage to unaffected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (*i.e*., the concentration of the test compound which achieves a half-maximal inhibition of symptoms) as determined in cell culture. Such information can be used to more accurately determine useful doses in humans. Levels in plasma can be measured by any technique known in the art, for example, by high performance liquid chromatography.

Furthermore, data obtained from patients after administration of a treatment regimen and its impact on disease-free survival and/or overall survival in comparable population may be used to determine the effective dose of a certain treatment regimen.

### 5.6. MONITORING THE EFFECT OF A THERAPEUTIC TREATMENT

The present application also describes a method for monitoring the effect of a selected therapeutic treatment regimen on a cancer patient during and after treatment.

Clinicians very much need a procedure that can be used to monitor the efficacy of cancer treatments. uPA and PAI-1 polypeptides and/or transcripts, other indicators or markers that are detectible in cancer patients, and particularly in breast cancer patients, can be used to measure disease regression in breast cancer patients. The therapeutic treatments which may be evaluated include but are not limited to radiotherapy, surgery, chemotherapy, vaccine administration, endocrine therapy, immunotherapy, and gene therapy, *etc*. The chemotherapeutic regimens include, but are not limited to administration of drugs such as, for example, methotrexate, fluorouracil, cyclophosphamide, doxorubicin, and taxol. The endocrine therapeutic regimens include, but are not limited to administration of tamoxifen, progestins, etc. A more detailed description of treatment methods are discussed *infra* in section 5.4.

The method comprises measuring at suitable time intervals before, during, or after therapy, the amount of uPA and PAI-1 transcripts or polypeptides, or other indicators or markers that are detectible in cancer patients. Any change or absence of change in the absolute or relative amounts of the uPA and PAI-1 gene products, or other indicators or markers that are detectible in cancer patients can be measured and correlated with the effect of the treatment on the subject.

In a preferred aspect, the approach that can be taken is to determine the levels of uPA and PAI-1 polyepeptide levels at different time points and to compare these values with a baseline level. The baseline level can be either the level of the uPA and PAI-1 polypeptides present in normal, disease-free individuals; and/or the levels present prior to treatment, or during remission of disease, or during periods of stability. These levels can then be correlated with the disease course or treatment outcome.

Other methods of detecting, monitoring and imaging cancer which may be used are discussed in Section 5.7.6 *infra.*

### 5.7. UROKINASE-TYPE PLASMINOGEN ACTIVATOR (UPA) AND PLASMINOGEN ACTIVATOR INHIBITOR TYPE-1 (PAI-1)

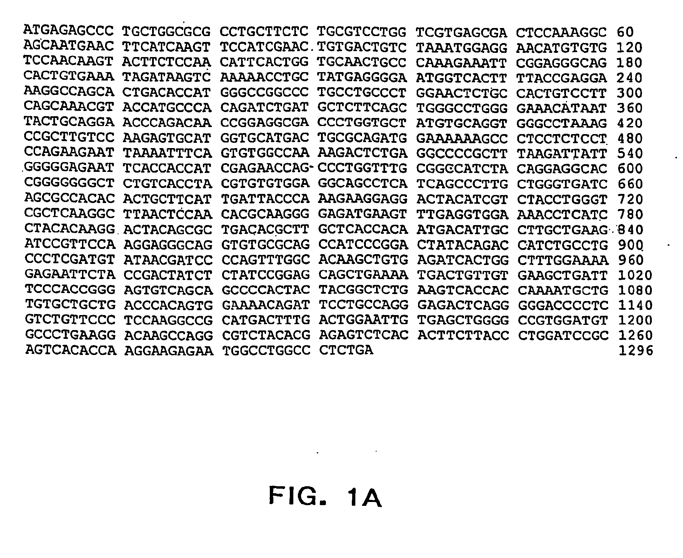

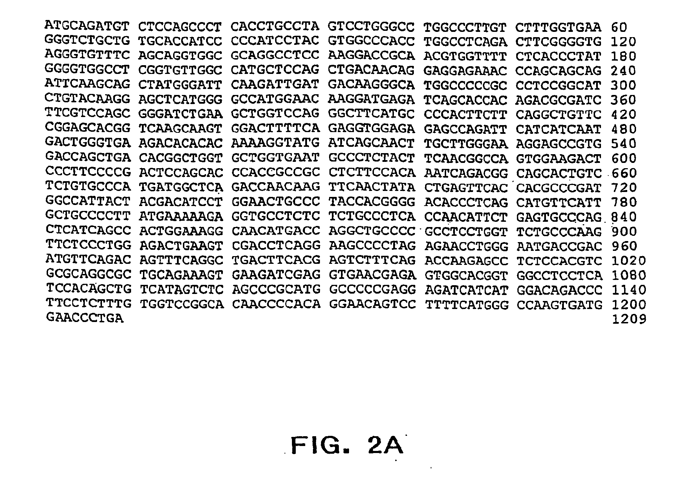

The methods of the present invention comprise measuring nucleic acid molecules that encode the uPA and PAI-1 proteins or their naturally occuring variants in subjects. According to the levels of uPA and PAI-1, the subjects are divided into high or low risk groups of cancer relapse. Different treatment regimes are implemented for subjects that belong to these two groups. Figure 1A shows the full-length uPA cDNA (1296 bp) (SEQ ID NO:1) with its amino acid sequence (431 a.a.) (SEQ ID NO: 2). Figure 2A shows the coding region of PAI-1 (1209 bp) (SEQ ID NO:3) and the amino acid sequence (402 a.a.) (SEQ ID NO: 4) that it encodes.

A nucleic acid molecule include DNA molecules (e.g., cDNA, genomic DNA), RNA molecules (e.g., hnRNA, pre-mRNA, mRNA), and DNA or RNA analogs generated using nuceotide analogs.

The invention includes the use of fragments or derivatives of any of the nucleic acid molecules disclosed herein. In various embodiments, a fragment or derivative comprises 10, 20, 50, 100, or 200 nucleotides, or multiple fragments thereof, that are complementary to the nucleic acid sequence of SEQ ID NO:1 or SEQ ID NO:3, or that its complement encodes all or a fragment of SEQ ID NO:2 or SEQ ID NO:4. In alternative embodiments, a nucleic acid is not more than 300, 1000, 2000, 5000, 7500, or 10,000 nucleotides in size.

The nucleic acid molecules that may be used in the present invention include but are not limited to the nucleic acids that are complementary to nucleic acid sequence of SEQ ID NO:1 or SEQ ID NO:3; a nucleic acid comprising a sequence hybridizable, under stringent condition as described above, to SEQ ID NO: or SEQ ID NO:3; or complementary to a nucleic acid at least 90% homologous to SEQ ID NO:1 and SEQ ID NO:3 (*e.g*., as determined using the NBLAST algorithm under default parameters).

The methods of the invention comprise measuring uPA and PAI-1 gene products in a sample derived from a subject. Levels of naturally occurring uPA and PAI-1 gene products, including, but not limited to wild-type uPA and PAI-1 gene products as well as mutants, allelic variants, splice variants, polymorphic variants, etc, may be measured. Isolated nucleic acid molecules may be used as probes to measure nucleic acid molecules that encode a variant protein or polypeptide. Such mutants and variants are highly homologous to SEQ ID NO:1 or SEQ ID NO:3, *e.g*., at least 90% homologous and/or hybridizable under high stringency conditions. In specific embodiments, the mutants and variants being measured comprise not more than 1, 2, 3, 4, or 5 point mutations (substitutions) compared to SEQ ID NO:1 or SEQ ID NO:3.

### 5.7.1. UPA AND PAI-1 PROTEINS FOR GENERATION OF ANTIBODIES

Antibodies that are generated against uPA and PAI-1, or peptide fragments thereof may be used to measure the levels of UPA and PAI-1 in a subject.

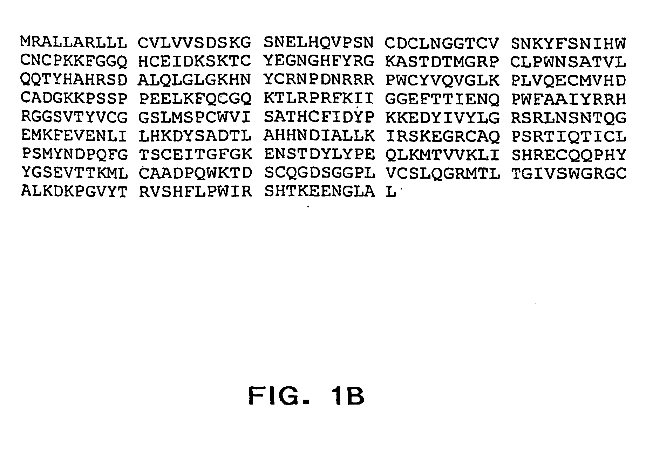

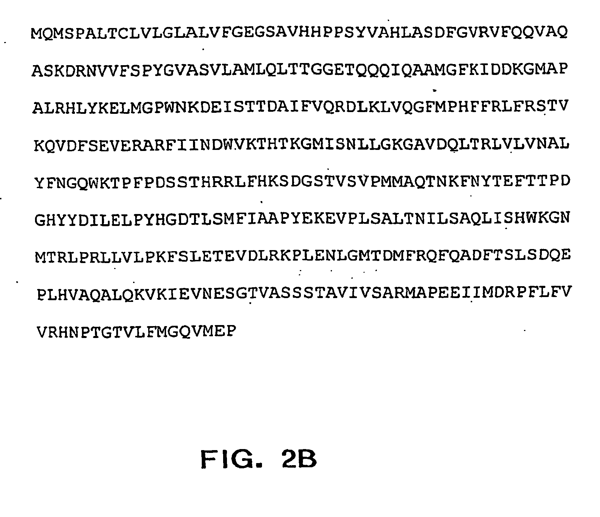

FIG. 1B shows the amino acid sequences of uPA (SEQ ID NO:2) and Figure 2B shows the amino acid sequence of PAI-1 (SEQ ID NO:4). The uPA and PAI-1 proteins and derivatives that may be used to generate antibodies used in the present invention include, but are not limited to proteins (and other molecules) comprising SEQ ID NO:2, SEQ ID NO:4, proteins comprising a sequence encoded by a nucleic acid hybridizable to SEQ ID NO:1 or SEQ ID NO:3 under high stringency condition, and proteins encoded by a nucleic acid at least 90% homologous to SEQ ID NO:1 or SEQ ID NO:3, *e.g*., as determined using the NBLAST algorithm. Due to the degeneracy of nucleotide coding sequences, other DNA sequences that encode substantially the same amino acid sequence as a component gene or cDNA can be used. The derivatives of a protein that may be used to generate antibodies used in the invention include, but are not limited to, those containing, an amino acid sequence, all or part of the amino acid sequence of the uPA or PAI-1 protein, including altered sequences in which functionally equivalent amino acid residues are substituted for residues within the sequence resulting in a silent change. For example, one or more amino acid residues within the sequence can be substituted by another amino acid of a similar polarity (a "conservative amino acid substitution") that acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

uPA and PAI-1 derivatives may include proteins that have conservative amino acid substitution(s) and/or display a functional activity of uPA and PAI-1 gene products. Such derivatives may contain deletions, additions or substitutions of amino acid residues within the amino acid sequence encoded by the uPA and PAI-1 gene sequences described, *infra,* in Section 5.7, but which result in a silent change, thus producing a functionally equivalent uPA and PAI-1 gene products.

The uPA and PAI-1 gene product sequences preferably comprises an amino acid sequence that exhibits at least 90% sequence similarity to uPA and PAI-1.

Protein comprising at least 10, 20, 30, 40 or 50 amino acids of SEQ ID NO:2 and SEQ ID NO:4, or at least 10, 20, 30, 40, 50, 75, 100, or 200 amino acids of SEQ ID NO:2 and SEQ ID NO:4 may be used to generate antibodies for use in the present invention. These proteins are capable of displaying one or more known functional activities associated with a full-length (wild-type) uPA or PAI-1 proteins. Such functional activities include but are not limited to antigenicity, ability to bind to its antibody, and immunogenicity (ability to generate antibodies).

### 5.7.2. ANTIBODIES TO uPA AND PAI-1 GENE PRODUCTS

The methods of the present invention encompass the use of antibodies or fragments thereof capable of specifically or selectively recognizing one or more uPA or PAI-1 gene product epitopes or epitopes of conserved variants. Such antibodies may include, but are not limited to, polyclonal antibodies, monoclonal antibodies (mAbs), humanized or chimeric antibodies, single chain antibodies, Fab fragments, F(ab')₂ fragments, Fv fragments, fragments produced by a Fab expression library, anti-idiotypic (anti-Id) antibodies, and epitope-binding fragments of any of the above. Such antibodies may be used to measure the level of uPA or PAI-1 gene product in a biological sample of a patient. The antibodies may also be included as a reagent in a kit for use in a method of the present invention.

In a specific embodiment, as further described in Section 5.4.3, antibodies generated against uPA and PAI-1 fragments, derivatives and analogs may be used to treat cancer.

Described herein are methods for the production of antibodies or fragments thereof Any of such antibodies or fragments thereof may be produced by standard immunological methods or by recombinant expression of nucleic acid molecules encoding the antibody or fragments thereof in an appropriate host organism.

For the production of antibodies against a uPA or PAI-1 gene product, various host animals may be immunized by injection with a uPA or PAI-1 gene product, or a portion thereof. Such host animals may include but are not limited to rabbits, mice, and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calinette-Guerin) and *Corynebacterium parvum.*

Polyclonal antibodies are heterogeneous populations of antibody molecules derived from the sera of animals immunized with an antigen, such as a uPA or PAI-1 gene product, or an antigenic functional derivative thereof. For the production of polyclonal antibodies, host animals such as those described above, may be immunized by injection with uPA or PAI-1 gene product supplemented with adjuvants as also described above.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, (1975, Nature 256:495; and U.S. Patent No. 4,376,110), the human B-cell hybridoma technique (Kosbor et al., 1983, Immunology Today 4:72; Cole et al., 1983, Proc. Natl. Acad. Sci. USA 80:2026), and the EBV-hybridoma technique (Cole et al., 1985, Monoclonal Antibodies And Cancer Therapy, Alan R. Liss, Inc., pp. 77). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers ofmAbs *in vivo* makes this the presently preferred method of production.

Techniques developed for the production of "chimeric antibodies" (Morrison et al., 1984, Proc. Natl. Acad. Sci., 81, 6851-6855; Neuberger et al., 1984, Nature 312, 604-608; Takeda et al., 1985, Nature 314, 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region. (See, *e.g*., Cabilly et al., U.S. Patent No. 4,816,567; and Boss et al., U.S. Patent No. 5,816,397). The invention thus contemplates chimeric antibodies that are specific or selective for the uPA or PAI-1 gene product.

Examples of techniques that have been developed for the production of humanized antibodies are known in the art. (See, *e.g*., Queen, U.S. Patent No. 5,585,089 and Winter, U.S. Patent No. 5,225,539.) An immunoglobulin light or heavy chain variable region consists of a "framework" region interrupted by three hypervariable regions, referred to as complementarity-determining regions (CDRs). The extent of the framework region and CDRs have been precisely defined (see, "Sequences of Proteins of Immunological Interest", Kabat, E. et al., U.S. Department of Health and Human Services (1983). Briefly, humanized antibodies are antibody molecules having one or more CDRs from the non-human species and framework regions from a human immunoglobulin molecule. The invention includes the use of humanized antibodies that are specific or selective for the uPA or PAI-1 gene product in the methods of the invention.

Phage display technology can be used to increase the affinity of an antibody to the uPA or PAI-1 gene product. This technique would be useful in obtaining high affinity antibodies to the uPA or PAI-1 gene product used in the method of the present invention. The technology, referred to as affinity maturation, employs mutagenesis or CDR walking and re-selection using the uPA or PAI-1 gene product antigen to identify antibodies that bind with higher affinity to the antigen when compared with the initial or parental antibody *(see, e.g.,*Glaser et al., 1992, J. Immunology 149:3903). Mutagenizing entire codons rather than single nucleotides results in a semi-randomized repertoire of amino acid mutations. Libraries can be constructed consisting of a pool of variant clones each of which differs by a single amino acid alteration in a single CDR and which contain variants representing each possible amino acid substitution for each CDR residue. Mutants with increased binding affinity for the antigen can be screened by contacting the immobilized mutants with labeled antigen. Any screening method known in the art can be used to identify mutant antibodies with increased avidity to the antigen (*e.g*., ELISA) (See Wu et al., 1998, Proc Natl. Acad Sci. USA 95:6037; Yelton et al., 1995, J. Immunology 155:1994). CDR walking which randomizes the light chain is also possible (*See* Schier et al., 1996, J. Mol. Bio. 263:551).

Alternatively, techniques described for the production of single chain antibodies (U.S. Patent 4,946,778; Bird, 1988, Science 242:423; Huston et al., 1988, Proc. Natl. Acad. Sci. USA 85:5879; and Ward et al., 1989, Nature 334:544) can be adapted to produce single chain antibodies against uPA or PAI-1 gene product. Single chain antibodies are formed by linking the heavy and light chain fragments of the Fv region via an amino acid bridge, resulting in a single chain polypeptide. Techniques for the assembly of functional Fv fragments in *E*. *coli* may also be used (Skerra et al., 1988, Science 242:1038).

As discussed in Section 5.4.3, the methods of therapy include using an antibody to a uPA or PAI-1 polypeptide, peptide or other derivative, or analog thereof that is a bispecific antibody (see generally, *e.g*., Fanger and Drakeman, 1995, Drug News and Perspectives 8:133-137) to treat cancer in a subject that expresses elevated levels of uPA or PAI-1 gene product. A bispecific antibody is genetically engineered to recognize both (1) an epitope and (2) one of a variety of "trigger" molecules, *e.g*., Fc receptors on myeloid cells, and CD3 and CD2 on T cells, that have been identified as being able to cause a cytotoxic T-cell to destroy a particular target. Such bispecific antibodies can be prepared either by chemical conjugation, hybridoma, or recombinant molecular biology techniques known to the skilled artisan.

Antibody fragments which recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science 246:1275-1281) to allow rapid and easy identification of monoclonal Fab fragments with the desired specificity.

### 5.7.3. MEASURING uPA & PAI-1 GENE PRODUCTS

The methods described herein may be performed, for example, by utilizing pre-packaged diagnostic test kits comprising at least one specific uPA and PAI-1 gene nucleic acid or anti-uPA and anti-PAI-1 antibodies, which may be conveniently used, *e.g*., in clinical settings or in home settings, to measure the levels of uPA and PAI-1 of patients, and to screen and identify those individuals that belong to a high risk group for treatment regimen and those that belong to a low risk group for another treatment regimen.

Nucleic acid-based detection techniques are described, below, in Section 5.7.4. Peptide detection techniques are described, below, in Section 5.7.5.

### 5.7.4. MEASURING uPA AND PAI-1 NUCLEIC ACID MOLECULES

The invention relates to methods for determining a treatment regimen for a subject by measuring quantitatively the levels of uPA and PAI-1 in a subject. Quantitative measurement may include measuring the exact amount of uPA or PAI-1 in a sample, or the relative amount of uPA or PAI-1 in a sample compared to a standard. Based upon the values, predictions can be made regarding disease-free survival and/or overall survival for a patient with or without a particular treatment. Using this information, a treatment regimen can be determined for the subject.

High risk subject is identified by high levels of both uPA and PAI-1, high level of uPA and low level of PAI-1 or, low level of uPA and high level of PAI-1. In an embodiment, uPA and PAI-1 levels may be measured in body fluid of a subject. Techniques well known in the art, *e.g*., quantitative RT PCR or Northern blot, can be used to measure the levels of uPA and PAI-1 in a subject. Castello et al., 2002, Clinical Chemistry 48(8): 1288-1295; Spyratos et al., 2002, Anticancer res. 22(5) 2997-3003; Noack et al., 1999, Int. J. Oncol. 15(4):517-23; and Luther et al., 2003, Throm. and Hemst (In press). Methods which describe quantitative measurement of uPA and PAI-1 levels in a subject are described in detail in the example *infra*. The measurement of uPA and PAI-1 levels can include measuring naturally occurring uPA and PAI-1 transcripts and variants thereof.

High level uPA is defined as above 3ng uPA/mg protein in primary tumor tissue extracts measured by ELISA. High level PAI-1 is defined as above 14ng PAI-1/mg protein. One of skill in the art may determine whether a subject has a high level of uPA or PAI-1 in any assay method by comparing a test sample with a standard sample with known uPA and PAI-1 levels, such as, at the respective cut off values of uPA and PAI-1. Such comparison places a test sample below, equal to, or above the cutoff values. Hence, the levels of uPA or PAI-1 can be standardized in different assay systems.

Treatment options for high risk subjects include, but are not limited to, adjuvant CMF chemotherapy, adjuvant non-CMF chemotherapy, adjuvant endocrine therapy, adjuvant adrianmycin chemotherapy, radiation therapy, and gene therapy. Other treatment options for high risk subjects may include therapies as discussed in Section 5.4.

Treatment options for low risk subjects include, but are not limited to, non-treatment and tamoxifen therapy. Other treatment options for low risk subjects may include therapies as discussed in Section 5.4.

In another example, RNA from a cell type or tissue known, to express the uPA and PAI-1 gene, such as breast cancer cells, or other types of cancer cells, including metastases, may be isolated and tested utilizing hybridization or PCR techniques as described, above. The isolated cells can be derived from cell culture or from a patient.

In one embodiment, a cDNA molecule is synthesized from a RNA molecule of interest by reverse transcription. All or part of the resulting cDNA is then used as the template for a nucleic acid amplification reaction, such as PCR or the like. The nucleic acid reagents used as synthesis initiation reagents (*e.g*., primers) in the reverse transcription and nucleic acid amplification steps of this method are chosen from among the uPA and PAI-1 gene nucleic acids described in Section 5.7. The preferred lengths of such nucleic acids are at least 9-30 nucleotides.

RT-PCR amplification techniques can be utilized to quantitatively measure the levels of uPA and PAI-1 transcripts in a subject. The levels of uPA and PAI- in a subject test sample may be calibrated against levels of uPA and PAI-1 in standard subjects with known levels of uPA and PAI-1. One of skill in the art may standardize uPA and PAI-1 levels in various assay methods so as to determine whether the test subject falls in the high risk or low risk group.

As an alternative to amplification techniques, standard Northern analyses can be performed. The preferred length of a probe used in a Northern analysis is 9-50 nucleotides. Utilizing such techniques, quantitative measurement of uPA and PAI-1 transcripts can also be determined.

Additionally, it is possible to measure the levels of uPA and PAI-1 *using in situ* assays, *i.e.,* directly upon tissue sections (fixed and/or frozen, e.g., paraffin sections) of patient tissue obtained from biopsies or resections, (e.g., laser micro-dissection of single cells) such that no nucleic acid purification is necessary. Noack et al., 1999, Int. J. Oncol. 15(4):617-23. Nucleic acid reagents such as those described in Section 5.7 maybe used as probes and/or primers for such *in situ* procedures (*see, e.g.,* Nuovo, G.J., 1992, PCR In Situ Hybridization: Protocols And Applications, Raven Press, NY).

### 5.7.5. MEASURING uPA AND PAI-1 PROTEINS

Antibodies directed against naturally occurring uPA and PAI-1, and naturally occurring variants thereof, which are discussed above, in Section 5.7.1, may be used in uPA and PAI-1 immunoassays.

The tissue or cell type to be analyzed will generally include those which are known, to express the uPA and PAI-1 gene, such as, for example, cancer cells including breast cancer cells, ovarian cancer cells, lymphoid cancer cells, and metastatic forms thereof. Preferably, excised primary breast cancer tumor. The protein isolation methods employed herein may, for example, be such as those described in Harlow and Lane (Harlow, E. and Lane, D., 1988, Antibodies: A Laboratory Manual, Cold Spring Harbor Laboratory Press, Cold Spring Harbor, New York).

For example, antibodies, or fragments of antibodies, such as those described above in Section 5.7.2, may be used to quantitatively measure uPA and PAI-1 polypeptides or naturally occurring variants thereof. The antibodies (or fragments thereof) useful in the present invention may, additionally, be employed histologically, as in immunofluorescence or immunoelectron microscopy, for *in situ* detection and quantitation of uPA and PAI-1 gene products or conserved variants thereof. *In situ* detection and quantitation may be accomplished by removing a histological specimen from a subject, such as paraffin embedded sections of tissue, *e.g*., breast tissues and applying thereto a labeled antibody of the present invention. The levels of uPA and PAI-1 may be measured quantitatively by counting the number of grains of label used on the sections. The antibody (or fragment) is preferably applied by overlaying the labeled antibody (or fragment) onto a biological sample.

Immunoassays for polypeptides or conserved variants thereof will typically comprise contacting a sample, such as a biological fluid, tissue or a tissue extract, freshly harvested cells, or lysates of cells which have been incubated in cell culture, in the presence of an antibody that specifically or selectively binds to uPA and PAI-1 gene product, *e.g*., a detectably labeled antibody capable of identifying uPA and PAI-1 polypeptides or conserved variants thereof, and detecting the bound antibody by any of a number of techniques well-known in the art (*e.g*., Western blot, ELISA, FACS).

In a specific embodiment, uPA and PAI-1 levels may be measured by the antigen levels of the analytes in primary tumor tissue extracts. In a preferred embodiment, the levels of uPA and PAI-1 are measured by any assay method. In a specific embodiment, a high level of uPA corresponds to levels above a cut-off value of at least about the 55^{th} percentile and no more than about the 75^{th} percentile of normalized uPA level for a randomized group of patients using any assay. In a specific embodiment, a high level of PAI-1 corresponds to PAI-1 levels above a cut-off value of at least about the 61^{st} percentile and no more than about the 81^{st} percentile of normalized PAI-1 levels for a randomized group of patients using any assay.

UPA and PAI-1 levels may also be measured by the antigen levels of analytes in primary tumor tissue extracts (not part of the claimed invention). Preferably, the levels of uPA and PAI-1 are measured by ELISA. High level uPA may be defined as above a cut-off value of at least about 2.4 ng/mg protein and no more than 4 ng uPA/mg protein. Similarly high level uPA may be defined as a cut-off value of above 3 ng uPA/mg protein. Specifically, high level PAI is defined as above a cut-off value of at least about 11 ng/mg protein and no more than 19 ng PAI-1/mg protein. Preferably, high level PAI-1 is defined as above a cut-off value of 14 ng PAI-1/mg protein.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled antibody that selectively or specifically binds to the uPA and PAI-1 polypeptides. The solid phase support may then be washed with the buffer a second time to remove unbound antibody. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, *etc.* Preferred supports include polystyrene beads. Those skilled in the art will know many other suitable carriers for binding antibody or antigen, or will be able to ascertain the same by use of routine experimentation.

The anti-uPA and anti-PAI-1 antibodies can be detectably labeled by linking the same to an enzyme and using the labeled antibody in an enzyme immunoassay (EIA) (Voller, A., "The Enzyme Linked Immunosorbent Assay (ELISA)", 1978, Diagnostic Horizons 2:1, Microbiological Associates Quarterly Publication, Walkersville, MD); Voller, A. et al., 1978, J. Clin. Pathol. 31:507-520; Butler, J.E., 1981, Meth. Enzymol. 73:482; Maggio, E. (ed.), 1980, Enzyme Immunoassay, CRC Press, Boca Raton, FL,; Ishikawa, E. et al., (eds.), 1981, Enzyme Immunoassay, Kgaku Shoin, Tokyo). The enzyme which is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Measurement of the levels of the proteins may be accomplished by visual comparison or electrical scanning calibrator of the extent of enzymatic reaction of a substrate in comparison with similarly prepared standards. Standards may be prepared from normal patient samples, or samples containing known uPA and PAI-1 levels or levels at or about the cutoff values for high risk and low risk subjects. Alternatively, standards containing known levels of uPA and PAI-1 may be used to calibrate the uPA and PAI-1 levels measured using various assay systems.

Levels of uPA and PAI-1 may also be measured using any of a variety of other immunoassays. For example, by radioactively labeling the antibodies or antibody fragments, it is possible to detect uPA and PAI-1 polypeptides through the use of a radioimmunoassay (RIA) (see, for example, Weintraub, B., Principles of Radioimmunoassays, Seventh Training Course on Radioligand Assay Techniques, The Endocrine Society, March, 1986). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, the amount of fluorescence can then be measured which indicates the level of the protein which the antibody binds. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody used in the present invention. Bioluminescence is a type of chemiluminescence found in biological systems in, which a catalytic protein increases the efficiency of the chemiluminescent reaction. The level of a bioluminescent protein is determined by detecting the amount of luminescence. Important bioluminescent compounds for purposes of labeling are luciferin, luciferase and aequorin.

The methods of the present invention involves the measurement of uPA and PAI-1 polypepeptides in the subject and is valuable in staging breast cancer and other cancers in a subject so that an appropriate therapeutic treatment regimen may be implemented on a subj ect.

In addition to the uPA and PAI-1 polypeptide at least one other marker, such as receptors or differentiation antigens can also be measured. For example, serum markers selected from, for example but not limited to, carcinoembryonic antigen (CEA), CA15-3, CA549, CAM26, M29, CA27.29 and MCA can be measured in combination with the uPA and PAI-1 polypeptides. Based upon the values, disease-free survival and/or overall survival for the patient without treatment or with a particular treatment may be predicted. Using this information, a treatment regimen may be selected for a subject. In another embodiment, the prognostic indicator is the observed change in different marker levels relative to one another, rather than the absolute levels of the markers present at any one time.

In a specific embodiment of the invention, uPA and PAI-1 polypeptides or in combination with other markers can be measured in any body fluid of the subject including but not limited to blood, serum, plasma, milk, urine, saliva, pleural effusions, synovial fluid, spinal fluid, tissue infiltrations and tumor infiltrates. In another embodiment the uPA and PAI-1 polypeptides are measured in tissue samples or cells directly. The present invention also contemplates a kit for measuring the levels of uPA and PAI-1 in a biological sample. The result is then used to place a subject in a high risk group or a low risk group where cancer treatment regimen specific to that group may be implemented. The kit may further comprise instructions for interpreting results and predicting overall survival and/or disease-free survival for a patient with or without particular breast cancer treatment after surgical removal of tumor tissue.

Any of numerous immunoassays can be used in the practice of the methods of the instant invention, such as those described in Section 5.7.5. Antibodies, or antibody fragments containing the binding domain, which can be employed include but are not limited to suitable antibodies among those in Section 5.7.2 and other antibodies known in the art or which can be obtained by procedures standard in the art such as those described in Section 5.7.2.

Any assay, such as those described in Section 5.7.5, can be used to measure the amount of uPA and PAI-1 polypeptides which measurements are compared to a baseline level. This baseline level can be the amount that is present in normal subject without cancer. The amount of uPA and PAI-1 polypeptides may also be compared to a known amount or an amount which is established to be the cutoff levels of uPA and PAI-1 in the tissue or body fluid of high risk and low risk subjects. An amount present in the tissue or body fluid of the subject which is higher than the cutoff level of uPA and PAI-1, higher level of uPA and lower level of PAI-1, or lower level of uPA and higher level of PAI-1, indicates that the subject is in a high risk group. An amount present in the tissue or body fluid of the subject which is lower than the cutoff level of uPA and PAI-1 indicates that the subject is in a low risk group.

### 5.7.6. IN VIVO IMAGING USING ANTIBODIES TO uPA AND PAI-1 POLYPEPTIDES

Current diagnostic and therapeutic methods make use of antibodies to target imaging agents or therapeutic substances, *e.g.*, to tumors. Thus, labeled antibodies specific or selective for the uPA or PAI-1 polypepeptide may be used for the *in vivo* imaging, measurement of uPA or PAI-1 levels, and treatment of cancer in a subject.

Antibodies may be linked to chelators such as those described in U.S. Patent No. 4,741,900 or U.S. Patent No. 5,326,856. The antibody-chelator complex may then be radiolabeled to provide an imaging agent for diagnosis or treatment of disease. The antibodies may also be used in the methods that are disclosed in U.S. Patent No. 5,449,761 for creating a radiolabeled antibody for use in imaging or radiotherapy.

In *in vivo* diagnostic applications, specific tissues or even specific cellular disorders, *e.g.*, cancer, may be imaged by administration of a sufficient amount of a labeled antibodies.

A wide variety of metal ions suitable for *in vivo* tissue imaging have been tested and utilized clinically. For imaging with radioisotopes, the following characteristics are generally desirable: (a) low radiation dose to the patient; (b) high photon yield which permits a nuclear medicine procedure to be performed in a short time period; (c) ability to be produced in sufficient quantities; (d) acceptable cost; (e) simple preparation for administration; and (f) no requirement that the patient be sequestered subsequently. These characteristics generally translate into the following: (a) the radiation exposure to the most critical organ is less than 5 rad; (b) a single image can be obtained within several hours after infusion; (c) the radioisotope does not decay by emission of a particle; (d) the isotope can be readily detected; and (e) the half-life is less than four days (Lamb and Kramer, "Commercial Production of Radioisotopes for Nuclear Medicine", In Radiotracers For Medical Applications, Vol. 1, Rayudu (Ed.), CRC Press, Inc., Boca Raton, pp. 17-62). Preferably, the metal is technetium-99m.

By way of illustration, the targets that one may image include any solid neoplasm, certain organs such breast, lymph nodes, parathyroids, spleen and kidney, sites of inflammation or infection (*e.g.*, macrophages at such sites), myocardial infarction or thromboses (neoantigenic determinants on fibrin or platelets), and the like evident to one of ordinary skill in the art.

As is also apparent to one of ordinary skill in the art, one may use *in vivo* therapeutics (*e.g.*, using radiotherapeutic metal complexes), especially after having diagnosed a diseased condition via the *in vivo* diagnostic method described above, or in *in vitro* diagnostic application (*e.g.*, using a radiometal or a fluorescent metal complex).

Accordingly, a method of measuring the levels of uPA and PAI-1 by obtaining an image of an internal region of a subject comprises administering to a subject an effective amount of an antibody composition specific or selective for uPA or PAI-1 polypeptide conjugated with a metal in which the metal is radioactive, and recording the scintigraphic image obtained from the decay of the radioactive metal. Likewise, it is possible to enhance a magnetic resonance (MR) image of an internal region of a subject which comprises administering to a subject an effective amount of an antibody composition containing a metal in which the metal is paramagnetic, and recording the MR image of an internal region of the subj ect.

Other methods include a method of enhancing a sonographic image of an internal region of a subject comprising administering to a subject an effective amount of an antibody composition containing a metal and recording the sonographic image of an internal region of the subject. In this latter application, the metal is preferably any non-toxic heavy metal ion. A method of enhancing an X-ray image of an internal region of a subject is also provided which comprises administering to a subject an antibody composition containing a metal, and recording the X-ray image of an internal region of the subject. A radioactive, non-toxic heavy metal ion is preferred.

### 5.8. KITS

Herein described is the use of a kit for measuring the levels of uPA and PAI-1 in a subject (*e.g.*, in a sample such as blood, urine, cell culture). The kit comprises a plurality of reagents, each of which is capable of binding specifically with a nucleic acid or polypeptide corresponding to uPA or PAI-1 genes or gene products or fragments thereof. Suitable reagents for binding with uPA or PAI-1 include antibodies, antibody derivatives, labeled antibodies, antibody fragments, and the like. Suitable reagents for binding with a nucleic acid (*e.g.*, a mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like. The kit also contains instructions for the diagnostic, prognostic and predictive methods of the invention.

The kit further comprises compositions for administration to a patient. Different compositions may be administered depending whether the patient is identified as high risk or low risk.

The kit may optionally comprise additional components useful for performing the methods of the invention. By way of example, the kit may comprise fluids (*e.g.*, SSC buffer) suitable for annealing complementary nucleic acids or for binding an antibody with a protein with which it specifically binds, one or more sample compartments, an instructional material for interpreting results and predicting treatment outcomes such as overall survival and/or disease-free survival for a patient.

### 6. EXAMPLES

### 6.1. MATERIALS AND METHODS

### 6.1.1. PATIENTS

The first study evaluated the clinical relevance of the combination of uPA and PAI-1 in 761 individual primary breast cancer patients (Table 3). Patients either underwent a modified radical mastectomy (n=389) or breast conserving surgery with subsequent breast irradiation (n=372) at the Department of Obstetrics and Gynecology, Technical University of Munich, Germany, between 1987 and 1998. Informed consent for analysis of tumor biological factors was obtained at primary surgery. Therapy decisions were based solely on consensus recommendations at the time but not on uPA and PAI-1. For 745 patients, information on adjuvant systemic therapy was available (Table 3). Median age of the patients at time of primary surgery was 56 years (range: 28-92 years). At time of primary therapy, no patient had any clinical or X-ray evidence of distant metastases. Median follow-up time of all patients still alive at time of analysis was 48 months (range: 1-142 months). Within the follow-up period, 194 patients (26 %) experienced disease recurrence and 164 patients (22 %) died. In addition to the collective as a whole, the subset of node-negative patients without adjuvant systemic therapy (n= 269; median follow-up 60 months) was analyzed separately (Table 3). For some patients, not all of the information was available. In addition to these node-negative patients, 12 node-positive patients (and 1 with unknown nodal status) did not receive adjuvant systemic therapy.

**Table 3: Patient characteristics of the first study: All patients presented with primary breast cancer without any evidence of distant disease.**

| **Prognostic factors** | | **All patients** (n=761) | **Node-negative patients** *without* adjuvant systemic therapy (n=269) |
|---|---|---|---|
| **Lymph node status** | Negative | 387 (51 %) | ------- |
| | Positive | 371 (48 %) | ------- |
| **Tumor size** | ≤2 cm | 305 (40 %) | 160 (60 %) |
| | > 2 cm | 446 (60 %) | 107 (40 %) |
| **Grade** | 1/2 | 414 (54 %) | 187 (70 %) |
| | 3 / 4 | 347 (46 %) | 82 (30 %) |
| **Steroid hormone receptor** | Negative | 151 (20 %) | 50(19%) |
| **status** | Positive | 608 (80 %) | 218 (81 %) |
| **Menopausal status** | Pre-/ perimenopausal | 270 (36 %) | 105 (39 %) |
| | Postmenopausal | 490 (64 %) | 163 (61 %) |
| **uPA/PAI-1** | Both low | 418 (55 %) | 171 (64 %) |
| | uPA high, PAI-1 low | 134 (17 %) | 40 (15%) |
| | uPA low, PAI-1 high | 81 (11%) | 23 (8%) |
| | Both high | 128 (17 %) | 35 (13%) |
| **Adjuvant systemic therapy** | None | 282 (38%) | 269 (100 %) |
| | Chemotherapy | 203 (27 %) | |
| | Hormone therapy | 201 (27 %) | |
| | Both | 59 (8%) | |

The second study involved a total of 3424 primary breast cancer patients from two different data sets. One set was collected from the Department of Obstetrics and Gynecology, Technical University of Munich, Germany, and the other was from the Department of Medical Oncology, Rotterdam Cancer Institute and University Hospital Rotterdam, the Netherlands. Patients had been treated for primary breast cancer between 1987 and 1999. Characteristics of the two data sets are summarized in Tables 4 and 5. Treatment decisions with regard to primary surgery and adjuvant systemic therapy were based primarily on consensus recommendations at the time. Treatment strategies differed between Munich and Rotterdam, particularly regarding administration of adjuvant systemic therapy to node-positive patients (Table 4b) (Harbeck et al., 2002, J. Clin. Oncology 20:1000-1009; Foekens et al., 2000, Cancer Res. 60:636-643). Of those patients treated by adjuvant chemotherapy, the majority received CMF (Rotterdam: 76 %, Munich: 65 %); about one-fifth in both collectives received anthracycline-containing regimens (Rotterdam: 24 %, Munich: 21 %); and the rest of the Munich patients were treated by other chemotherapy regimens. The endocrine treatment used in both collectives was tamoxifen.

Median age of the patients at time of primary surgery was 56 years (range: 22-94 years). Both data sets contained established clinical and histo-morphological factors such as number of involved axillary lymph nodes, pT stage, estrogen receptor status, progesterone receptor status, exposure to adjuvant chemotherapy (CT), adjuvant endocrine therapy (HT), or adjuvant radiotherapy (RT), as well as uPA and PAI-1 measurements. Since the scoring systems for assessment of grade differed substantially between the centers and could thus represent different biological tumor characteristics, grade was not included in the combined stratified analysis. The variables were re-coded as described below in Section 6.1.3. Patients with an uncertain number of affected lymph nodes were included for computing the univariate distributions of ranked variables but not in multivariate survival analysis. Forty-five Rotterdam patients with an uncertain number of affected lymph nodes and three Munich patients with missing information on chemotherapy were not included. At the time of primary therapy, no patient had any clinical or radiological evidence of distant metastases. Follow-up data were obtained at regular intervals. Median follow-up time of all patients still alive at time of analysis was 83 months (range: 1-183 months). Within the follow-up period, 1319 patients (39 %) experienced disease recurrence and 1200 patients (35 %) died.

**Table 4: a) Overview of patient and treatment characteristics in the second study b) Adjuvant endocrine and chemotherapy by nodal status and center**

| **Factors** | | **Munich** | **Rotterdam** | **Total** |
|---|---|---|---|---|
| **Tumor size** | **pT** 1 | 289 (38%) | 1128 (42 %) | 1417 (41%) |
| | **pT 2** | 360 (47%) | 1246 (47%) | 1606 (47%) |
| | **pT 3 / 4** | 118 (15%) | 283(11%) | 401(12%) |
| **Involved lymph nodes** | **0** | 399 (52%) | 1337 (50%) | 1736 (51%) |
| | **1-3** | 189 (25 %) | 668 (25%) | 857 (25 %) |
| | **≥ 4** | 179 (23 %) | 607 (23%) | 786 (24 %) |
| | **uncertain** | 0 | 45 (2 %) | 45 (1 %) |
| **uPA/PAI-1** | **low** | 414 (54%) | 1427(54%) | 1841 (54%) |
| | **high** | 353 (46 %) | 1230 (46 %) | 1583 (46%) |
| | | | | |

| | **Munich** | | **Rotterdam** | |
|---|---|---|---|---|
| **Adjuvant therapy** | N0 | N1 | N0 | N1 |
| **None** | 271 (67.9 %) | 12 (3.3%) | 1337 (100%) | 663 (52.0 %) |
| **endocrine only** | 69 (17.3 %) | 140 (38.4 %) | 0 | 161 (12.6 %) |
| **chemo only** | 56 (14.0 %) | 160 (43.8 %) | 0 | 430 (33.7 %) |
| **chemo and endocrine** | 3 (0.8%) | 53 (14.5%) | 0 | 21 (1.6%) |

**Table 5: Frequency of adjuvant systemic therapy in data sets by uPA / PAI-1 levels.**

| **Adjuvant systemic therapy by uPA/PAI-1** | **Munich** | | **Rotterdam** | |
|---|---|---|---|---|
| | **uPA *l* PAI-1** | | **uPA / PAI-1** | |
| | **low** | **high** | **low** | **High** |
| **none** | 179 (43 %) | 104 (30%) | 1086 (76 %) | 945 (77 %) |
| **endocrine only** | 106 (26 %) | 103 (29%) | 83 (6 %) | 85 (7 %) |
| **chemo only** | 96 (23%) | 120 (34 %) | 243 (17%) | 192 (16 %) |
| **chemo and endocrine** | 32 (8 %) | 24 (7 %) | 15 (1%) | 8 (1%) |

### 6.1.2. LABORATORY ASSAYS

In studies involving patients from Munich, uPA and PAI-1 antigen have been prospectively measured by ELISA (uPA: Imubind # 894. PAI-1: Imubind # 821; both from American Diagnostica Inc., Greenwich, CT) since 1987 in all primary breast cancer patients treated at our institution. (Jänicke et al., 1994, Cancer Res. 54: 2527-2530). The antigen levels in detergent extracts of breast cancer tissue are expressed as ng of analyte per mg of tissue protein. In the study involving Rotterdam patients, uPA and PAI-1 antigen were measured by ELISA, employing the same antibodies as above, in cytosol preparations of the primary tumor as described by Foekens et al., 2000, Cancer Res., 60:636-643.

Tumor grade was determined using the well established Bloom-Richardson criteria. Steroid hormone receptors (estrogen and progesterone receptors) were initially determined biochemically (EIA) in cytosol fractions and considered positive if they contained at least 20 fmol per mg protein. Starting in 1991, immunohistochemical staining on paraffin-embedded tissue sections was performed; positive staining denoted receptor positivity. Steroid hormone receptor status was considered positive if either or both receptors were positive.

### 6.1.3. STATISTICAL ANALYSES

The continuous variables uPA and PAI-1 were first coded as binary variables using the previously optimized and re-evaluated cutoffs of 3 ng uPA / mg protein and 14 ng PAI-1 / mg protein to distinguish between high and low antigen levels of the analytes in primary tumor tissue extracts. Harbeck et al., 1999, Breast Cancer Res Treat 54: 147-157. A new binary variable "uPA/PAI-1 ", representing the combination of these two factors, was then defined as both factors low vs. either or both factors high.

Due to differences in measurement techniques between the Munich and Rotterdam data sets, data re-coding was required. For the laboratory measurements of uPA, PAI-1, Estrogen receptor, and progesterone receptor, fractional ranks were computed with respect to each distribution. Fractional ranks also keep the variables on a convenient scale from zero to one, thus facilitating comparison of the β coefficients of different factors. In particular, ranked estrogen receptor and progesterone receptor measurements were included as continuous variables in the statistical models, even though biochemical and immunohistochemical assays were used. The weak correlations found between these laboratory measurements and the remaining "classical" staging factors support the inference that similar ranks imply similar biological characteristics even across data sets.

A binary variable for uPA/PAI-1 was defined 0 for uPA and PAI-1 both below their respective cutoffs and 1 otherwise (i.e., either or both above the respective cutoff) (Harbeck et al., 1999, Breast Cancer Res. Treat. 54:147-157). Previously determined and validated univariate cutoff values by the Munich group (Jänicke et al., 2001, Int. J. Natl. Cancer Inst. 93: 913-920; Harbeck, et al., 1999, Breast Cancer Res. Treat., 54:147-157) were applied to the Rotterdam data by transforming the Munich cutoffs to fractional ranks and applying these to the Rotterdam data, resulting in almost exactly the same percentage of uPA/PAI-1 "high" vs. "low" in each cohort (see Table 4a).

The pT stage (Harris, 2000, "Staging and natural history of breast cancer," in Diseases of the Breast, 2nd ed., Harris, eds., 403-406) was coded using two auxiliary binary variables: 1) pT1 (coded 0) vs. all others (coded 1), and 2) pT1 and 2 (coded 0) vs. pT 3 and 4 (coded 1). Fractional ranks were assigned separately within the two data sets for the number of affected lymph nodes (variable denoted "lymph nodes"). Equal numbers of nodes correspond to equal fractional ranks across data sets, to within a few percent. For patient age, fractional ranks were first computed for the Rotterdam data set, and the Munich ages were then transformed to this scale. In order to model the nonlinear dependence of HR on age as closely as possible, both the fractional rank itself as well as its square is included in the models. The three binary variables for adjuvant therapy (RT, HT, CT) were coded such that the value 1 represents "known to have been treated by the respective kind of adjuvant therapy." A binary variable "data set" was introduced and used to stratify the analysis as discussed below. This variable accounts for systematic differences in demographic influences, unobserved factors contributing to the stage of the disease, or adjuvant systemic therapy strategies.

Established prognostic factors were dichotomized as described elsewhere. Harbeck et al., 1999, Br. J. Cancer 80: 419-426. For univariate analysis of disease-free (DFS) and overall survival (OS), Kaplan-Meier curves were plotted and then compared using log-rank statistics. Multivariate analyses were performed in a stepwise forward fashion by applying the Cox proportional hazards model and Cox models with time varying covariates using the SPSS software package (SPSS Inc., Chicago, IL). Interactions were included within the Cox models in the second stage of the model using forward selection. The Cox proportional hazards model was used with continuous ranked variables and binary variables as described above. Variables were included according to likelihood ratios in a stepwise forward fashion using the SPSS software package (SPSS Inc., Chicago, IL). Unless otherwise stated, main (i.e., linear) effects were always included as a first block, while interactions were included as a second block in the analysis. This method implies that a main effect that is significant in the first block will be retained in the model, even if an interaction in the second block is so strong as to reduce the main effect coefficient below the level of significance. All models were stratified by data set. The SPSS software package was also used to compute fractional ranks, correlation coefficients, associations, and other statistical properties. All tests were performed at a significance level of α = 0.05. Confidence intervals refer to the 95 % level. Significant correlations (Pearson's coefficient) exceeding 0.1 between ranked factors were found between estrogen receptor and progesterone receptor (0.53); estrogen receptor and age (0.36); lymph nodes and tumor stage (0.36); and uPA and PAI-1 (0.58).

### 6.2. RESULTS

### 6.2.1. COMBINATION UPA/PAI-1 IDENTIFIES LOW-RISK PATIENTS BETTER THAN EITHER FACTOR ALONE

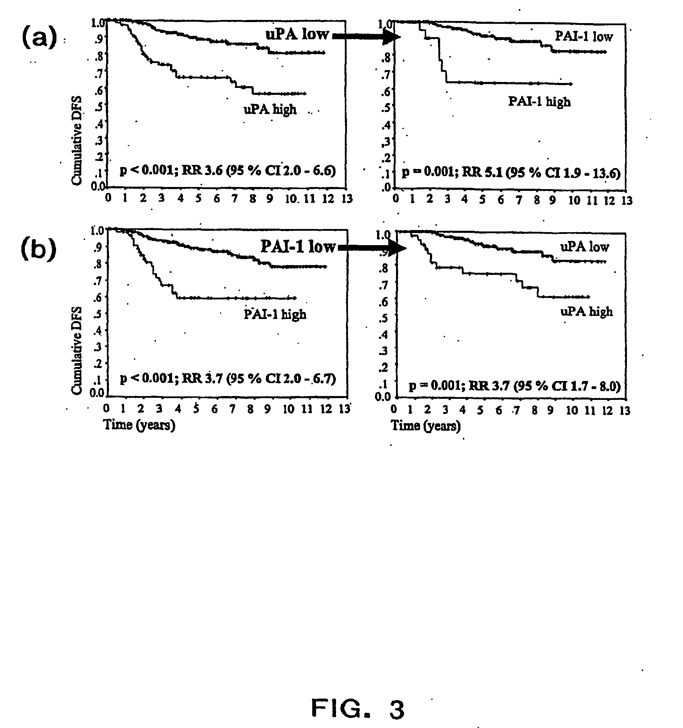

In order to address the question of improved risk group discrimination by the combination of uPA and PAI-1, node-negative patients without adjuvant systemic therapy were examined since in this subset the prognostic impact reflects the natural course of the disease. Both uPA (p=0.022; RR 2.3; 95 % CI 1.1- 4.1) and PAI-1 (p=0.049; RR 2.0; 95 % CI 1.0 - 4.0) separately as well as grade (p=0.026; RR 2.1; 95 % CI 1.1 - 4.0) are significant in multivariate Cox regression for DFS (including established factors tumor size, grade, hormone receptor and menopausal status). However, if the dichotomized combination uPA/PAI-1 (low-low vs. either or both high) is entered into Cox regression on this cohort, then uPA and PAI-1 both drop out of the model. In order to understand the special role of the combination uPA/PAI-1, each of the two factors were stratified and a Cox regression was performed on the remaining factors in the respective low-risk subgroup: In patients with low uPA, only PAI-1, but none of the established factors, provides additional risk discrimination (p<0.001; RR 5.9; 95 % CI 2.2 -16.0). Similarly, in patients with low PAI-1, uPA provides additional prognostic information (p=0.001; RR 4.2; 95 % CI 1.9 - 9.6) even in multivariate analysis. This behavior is illustrated by the respective Kaplan-Meier curves in Figure 3. As shown in the upper panels, PAI-1 provides statistically significant risk group separation (PAI-1 low: n=171, 13 events; PAI-1 high: n=23, 6 events) inpatients considered low-risk by uPA levels in their primary tumor tissue (uPA low: n=194, 19 events; uPA high: n=75, 25 events). In the lower panels, it is seen that uPA provides statistically significant risk group separation (uPA low: 171 patients, 13 events; uPA high: 40 patients, 12 events) in patients considered low-risk according to PAI-1 (PAI-1 low: n=211, 25 events; PAI-1 high: n=58, 19 events). Interestingly, the influence of either of these two factors is not uniform with respect to the other, but is significant only in the *low-risk* subgroup of the other.

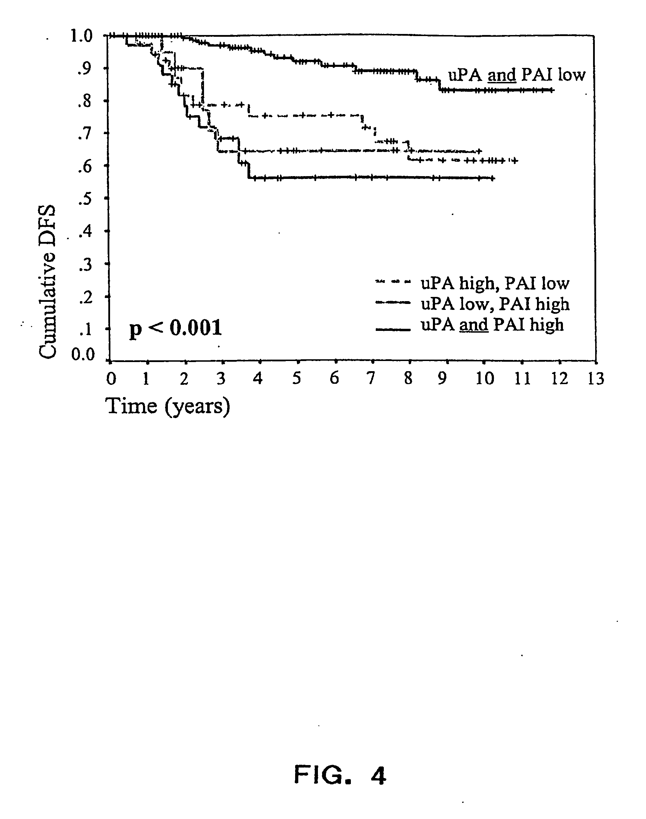

Figure 4 shows the respective Kaplan-Meier curves for all four possible combinations of both factors. Low levels of both uPA and PAI-1 (n=171, 13 events) identify low-risk patients and significantly outperform all other combinations (uPA high, PAI-1 low: n=40, 12 events; uPA low, PAI-1 high: n=23, 6 events; uPA and PAI-1 high: n=35, 13 events). The relapses in patients with high uPA or PAI-1 or both tend to occur within the first 3-4 years, especially if PAI-1 is high. The diminished prognostic impact with time, which is also found in other well-known prognostic factors, suggests a departure from strictly "proportional hazards." In order to model this behavior more closely, a time-variation F(T) was included in describing the interaction of the factors uPA and PAI-1. A logistic form F(T)= 1/(1 +EXP((T-36)/6), is used, where T is the time in months. This functional form allows the contribution of uPA/PAI-1 to remain strong through about 3 years and then rapidly diminish toward zero with longer follow-up; the precise form of F(T) affects the fit but otherwise makes little qualitative difference in understanding the interaction. Models were constructed including all dichotomized established factors and dichotomized uPA*F(T), PAI-1 *F(T), the *interaction term* uPA*(PAI-1)*F(T) as well as these factors without the time-dependence. The model with the best fit includes only grade, uPA*F(T), PAI-1 *F(T), and uPA*(PAI-1)*F(T). The beta coefficients of all three terms are the same in magnitude, about 2.7 (corresponding to a relative risk of about 15), but the coefficient of the interaction is *negative.* This result means that the log relative risk (R.R.) associated with the combination of high uPA and high PAI-1 is not twice the log relative risk as would be expected from a linear model, but is close to that for either factor alone. These relationships are reflected in FIG. 4. These results support the statement that the particular combination uPA/PAI-1 as used here (either or both high vs both low) correctly characterizes the risk associated with uPA and PAI-1.

### 6.2.2. PROGNOSTIC IMPACT OF UPA/PAI-1 IN NODE-NEGATIVE PATIENTS WITHOUT ADJUVANT THERAPY

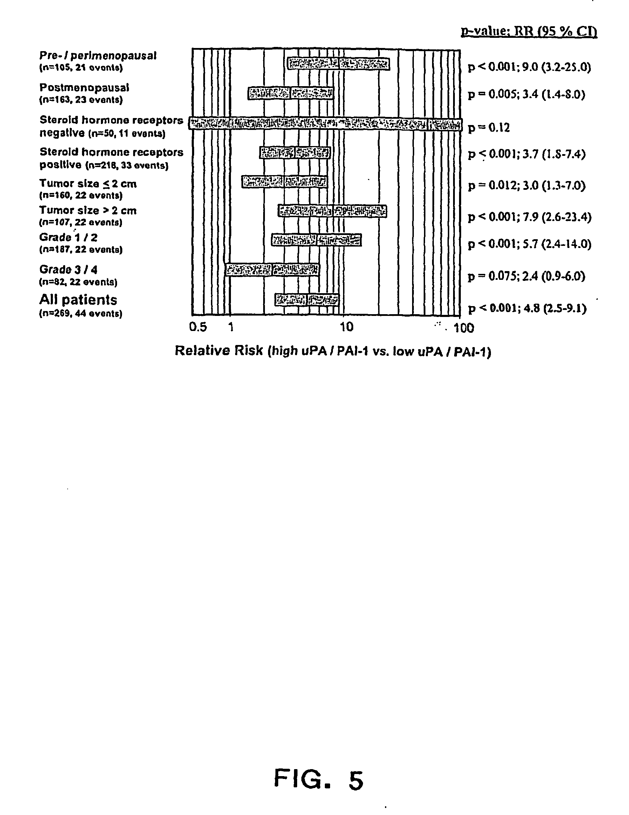

The combination uPA/PAI-1 is a highly significant discriminator between patients at low and those at high risk not only for relapse but also for death in univariate analysis in this clinically relevant subgroup. In multivariate analysis, uPA/PAI-1 is the strongest prognostic factor not only for DFS but also for OS (Table 6). Moreover, uPA/PAI-1 enables identification of high-risk patients even within established risk groups defined by tumor size, grade, steroid hormone receptor or menopausal status: In Figure 5, relative risk (RR) of recurrence is given as a function of high antigen levels of either or both factors vs. low levels of both uPA and PAI-1 as determined in primary tumor tissue extracts.

**Table 6: Univariate and multivariate analyses for disease-free and overall survival in node-negative breast cancer patients without adjuvant systemic therapy (n=269; median follow-up time 60 months).**

| **Prognostic factors** | **Disease-free survival (DFS)** | | | | **Overall survival (OS)** | | | |
|---|---|---|---|---|---|---|---|---|
| | Univariate p-value | relative risk (95 % Confidence interval) | multi-variate p-value | Relative risk (95 % Confidence interval) | univariate p-value | relative risk (95 % Confidence interval) | multi-variate p-value | relative risk (95 % Confidence interval) |
| **uPA/PAI-1** | < 0.001 | 4.8 (2.5 - 9.1) | < 0.001 | 3.9 (2.0 - 7.5) | < 0.001 | 3.9 (1.9 - 7.8) | 0.005 | 2.8 (1.4-5.9) |
| **Grade** | < 0.001 | 3.3 (1.8 - 5.9) | 0.007 | 2.3 (1.3 - 4.3) | < 0.001 | 3.5 (1.8 - 6.8) | 0.003 | 2.8 (1.4 - 5.6) |
| **Menopausal status** | n.s. ^{§} | - | n.s. | - | 0.047 | 2.2 (1.9 - 4.9) | 0.037 | 2.3 (1.1 - 5.1) |
| **Steroid hormone receptor Statues** | n.s. | - | n.s. | - | 0.022 | 0.5 (0.2 - 0.9) | n.s. | - |
| **Tumor size** | n.s. | - | n.s. | - | n.s. | - | n.s. | - |

| | | | | | | | | |
|---|---|---|---|---|---|---|---|---|
| § n.s. = not significant (p > 0.05) | | | | | | | | |

### 6.2.3. PROGNOSTIC IMPACT OF UPA/PAI-1 IN THE WHOLE PATIENT COLLECTIVE

In multivariate analysis of the *whole collective* (n=761) of primary breast cancer patients, uPA/PAI-1 emerges as the strongest statistically independent prognostic factor for disease-free (DFS) and overall (OS) survival next to nodal status (Table 7). In addition, uPA/PAI-1 provides significant risk group separation even within clinically important subgroups as stratified by established prognostic factors. For the following subgroups, relative risks of recurrence are given as a function of high uPA/PAI-1 vs. low uPA/PAI-1: Nodal status (negative RR 3.8; 95 % CI 2.1-6.8. positive: RR 1.5; 95 % CI 1.1-2.1), tumor size (≤2cm: RR 1.9; 95 % CI 1.1-3.4. > 2 cm: RR 1.8; 95 % CI 1.3-2.6), grade (G 1/2: RR 2.3; 95 % CI 1.5-3.6), hormone receptor status (positive: RR 1.8; 95 % CI 1.3-2.5), and menopausal status (pre/peri: RR 2.8; 95 % CI 1.8-4.4. post: RR 1.5; 95 % CI 1.1-2.2).

**Table 7: Univariate and multivariate analyses for disease-free and overall survival in patients with primary breast cancer (n=761), median follow-up time 48 months).**

| **Prognostic factors** | **Disease-free survival (DFS)** | | | | **Overall survival (OS)** | | | |
|---|---|---|---|---|---|---|---|---|
| | Univariate p-value | relative risk (95% Confidence interval) | multi-variate p-value | Relative risk (95% Confidence interval) | univariate p-value | relative risk (95% Confidence interval) | multi-variate p-value | relative risk (95% Confidence interval) |
| **Lymph node status** | < 0.001 | 2.7 (2.0 - 3.7) | < 0.001 | 3.7 (2.3 - 5.8) | < 0.001 | 2.6 (1.8 - 3.6) | <0.001 | 2.3 (1.6-3.2) |
| **uPA/PAI-1** | < 0.001 | 1.9 (1.4 - 2.5) | < 0.001 | 1.9(1.4 - 2.5) | < 0.001 | 2.0 (1.5 - 2.7) | <0.001 | 2.0 (1.4 - 2.7) |
| **Tumor size** | < 0.001 | 2.2 (1.6 - 3.0) | 0.006 | 1.6 (1.2 - 3.0) | < 0.001 | 2.2 (1.5 - 3.2) | 0.032 | 1.5(1.0 - 2.2) |
| **Grade** | < 0.001 | 2.3 (1.7 - 3.0) | 0.004 | 1.6 (1.2 - 2.2) | < 0.001 | 2.2 (1.6 - 3.1) | 0.004 | 1.6 (1.2 - 2.4) |
| **Adjuvant endocrine therapy** | n.s.§ | - | 0.001 | 0.5 (0.3 - 0.7) | n.s. | - | n.s. | - |
| **Adjuvant chemotherapy** | < 0.001 | 1.7 (1.3 - 2.3) | 0.019 | 0.6 (0.4 - 0.9) | n.s. | - | n.s. | - |
| **Steroid hormone receptor Status** | 0.008 | 0.6 (0.5 - 0.9) | n.s. | - | 0.001 | 0.6 (0.4 - 0.8) | n.s. | - |
| **Menopausal status** | n.s. | - | n.s. | - | n.s. | - | n.s. | - |

| | | | | | | | | |
|---|---|---|---|---|---|---|---|---|
| ^{§} n.s. = not significant (p > 0.05) | | | | | | | | |

### 6.2.4. INTERACTION OF ADJUVANT SYSTEMIC THERAPY WITH PROGNOSTIC IMPACT OF UPA/PAI-1

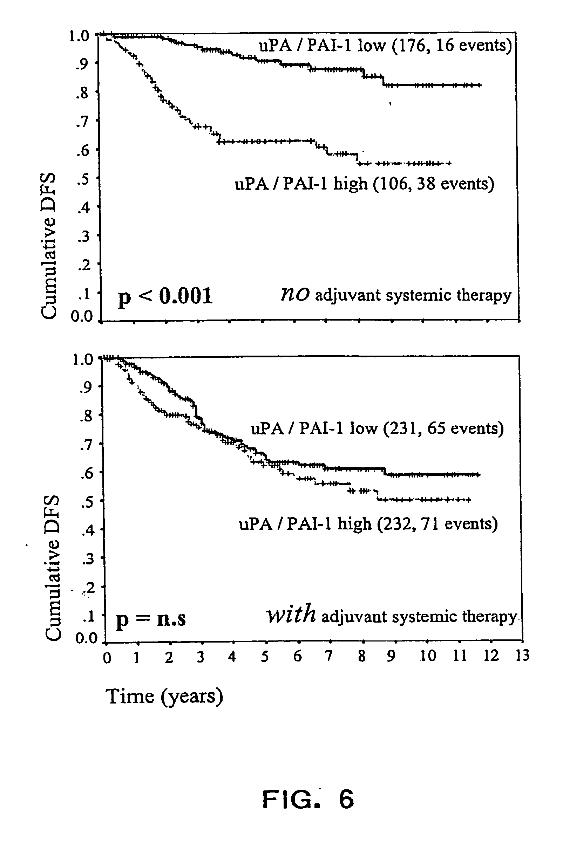

The prognostic impact of uPA/PAI-1 greatly depends on administration of adjuvant systemic therapy. In patients who did not receive any adjuvant systemic therapy, uPA/PAI-1 allows highly significant discrimination between patients at low risk and those at high risk for disease recurrence (p<0.001; RR 4.6; 95 % CI 2.6-8.3) (Figure 6, upper panel). In patients who received adjuvant systemic therapy, the prognostic significance is lost (p=0.165; RR 1.3; 95 % CI 0.9-1.8) (Figure 6, lower panel). This remains true even if one distinguishes between adjuvant chemotherapy (p=0.260; RR 1.3; 95 % CI 0.8-2.2) or adjuvant endocrine therapy (p=0.404; RR 1.3; 95 % CI 0.7-2.2) separately.

Table 8 presents the results of a Cox model including all dichotomized established factors, uPA/PAI-1, a dichotomized therapy variable (adjuvant systemic treatment yes/no), the interaction between the therapy variable and uPA/PAI-1, as well as a hypothetical interaction of nodal status with uPA/PAI-1. The interactions were included in the second stage of the model using forward selection. In the first (linear) stage, nodal status, tumor size, grade, uPA/PAI-1, and "therapy" are all significant. After the second stage, however, the interaction are considered, the interaction between the therapy variable and uPA/PAI-1 enters the model, with therapy alone losing its significance. The hypothetical interaction of nodal status with uPA/PAI-1 does not enter this model. However, in similar models in which the therapy interaction is not included, the nodal status interaction does enter. This statistical effect is consistent with the strong confounding of nodal status and adjuvant treatment status. For OS, uPA/PAI-1 again shows a significant prognostic impact in patients without adjuvant systemic therapy (p<0.0001; RR 3.8; 95 % CI 2.1-7.2), whereas its prognostic strength is diminished in patients who received adjuvant chemotherapy (p=0.023; RR 2.0; 95 % CI 1.1-3.7) or adjuvant endocrine therapy (p=0.467; RR 1.2; 95 % CI 0.7-2.0).

**Table 8: Multivariate Cox model (DFS) including interaction of uPA/PAI-1 with treatment in primary breast cancer. First stage of analysis included established prognostic factors, uPA/PAI-1, and adjuvant systemic therapy (yes vs no). Second stage included interactions: Adjuvant therapy with uPA/PAI-1, and nodal status with uPA/PAI-1.**

| **Factors** | **Final model** | |
|---|---|---|
| | p-value | Relative Risk (95% Confidence interval) |
| **Lymph node status** | < 0.001 | 4.5 (2.5 - 8.2) |
| **uPA/PAI-1** | < 0.001 | 4.3 (2.4 - 7.8) |
| **Tumor size** | 0.003 | 1.7 (1.2 - 2.4) |
| **Grade** | 0.002 | 1.7 (1.2 - 2.2) |
| **Adjuvant systemic therapy** | 0.404^{§} | 0.7 (0.3 -1.5) |
| **Interaction "therapy," uPA/PAI-1"** | 0.001 | 0.3 (0.2 - 0.6) |

| | | |
|---|---|---|
| ^{§} not significant, kept in model as "main effect" | | |

### 6.2.5. DFS INCLUDING UPA/PAI-1 AND THEIR INTERACTIONS WITH THERAPY IN ALL PATIENTS

The 5-year relapse rates associated with low and high uPA/PAI-1 were 28 % and 46 %, respectively. The probabilities of being treated by CT or HT in subgroups defined by uPA/PAI-1 are depicted in Table 5 in Section 6.1.

In Table 9, the results of a proportional hazards analysis for DFS in all patients, stratified by data set are reported. The first stage included established prognostic factors (estrogen receptor, progesterone receptor, age, lymph nodes, pT stage, coded as described above under Section 6.1.3.) as well as uPA/PAI-1, exposure to CT, HT, or RT. The second stage included the interactions CT and HT with uPA/PAI-1, and lymph nodes with uPA/PAI-1, as well as the "treatment interaction", i.e., CT with HT. For this analysis, 45 out of a total of 3424 patients were excluded due to incomplete information on number of involved nodes, and three were excluded due to missing information on adjuvant chemotherapy. Significant factors were coded as reported in Section 6.1. Analysis was also stratified by center: 46 patients were censored before first event in stratum. There were 1301 events total. First stage of analysis included established prognostic factors, uPA/PAI-1, adjuvant radiotherapy, adjuvant chemotherapy, and adjuvant endocrine therapy. Second stage included interactions: Chemo- and endocrine therapy with uPA/PAI-1, chemotherapy with endocrine therapy, and involved lymph nodes with uPA/PAI-1.

All of the main effects (factors) considered in the model of Table 9 are significant except radiotherapy and Estrogen receptor. The HR (hazard ratio) of uPA/PAI-1 is 2.0 (1.8-2.3), (P < 0.001). The overall effect of age - including squared fractional rank - in the model is a gradual, almost linear drop of the HR from the youngest patients (defined to have HR =1), leveling off to about HR = 0.5 by about age 60 and apparently rising slightly above about age 65. The interaction between lymph node involvement and uPA/PAI-1 was not significant in the analysis of all patients, nor was CT * HT, implying no evidence against an additive effect of this treatment combination.

The key result is the significant (negative) interaction between CT and the variable uPA/PAI-1. This interaction implies that the higher HR of relapse (2.01) associated with high uPA/PAI-1 (compared to low uPA/PAI-1) is significantly reduced (0.68*2.01 = 1.36) in patients who receive adjuvant chemotherapy. This benefit occurs in addition to the independent overall risk reductions of about one-third due to CT (HR = 0.69) - or HT (HR = 0.68). No significant interaction was found between HT and uPA/PAI-1 (95 % CI for this HR: 0.66 - 1.28). Hence, the benefits of both therapies are significant, but only for chemotherapy is an additional (enhanced) benefit seen among high uPA/PAI-1 patients.

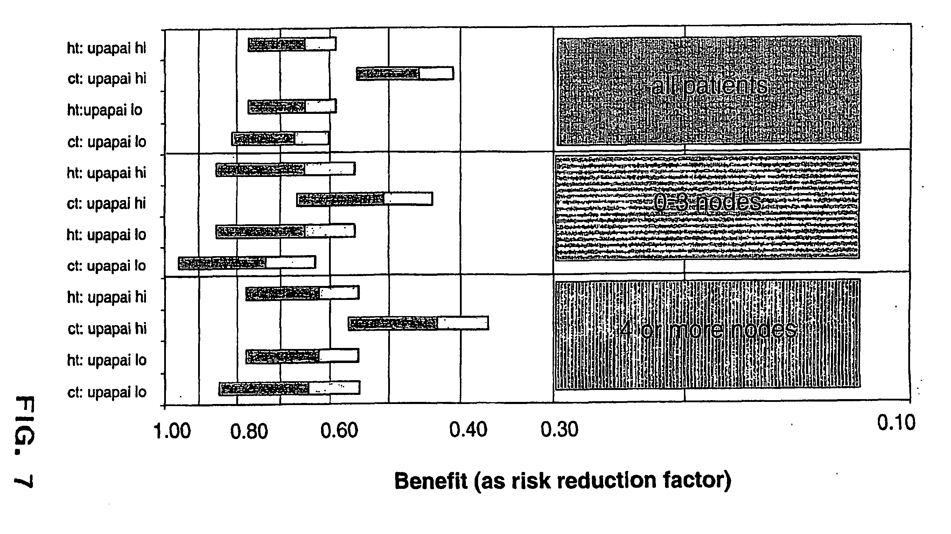

FIG. 7 illustrates the HRs of CT and HT taking into account significant interactions with uPA/PAI-1 according to Tables 9-11 (for discussion of Tables 10 and 11, see following sections). For all patients, the significant interaction CT * uPA/PAI-1 is seen in the upper panel of FIG. 7 as a hazard reduction attributable to CT that is strongly affected by uPA/PAI-1. The lack of a significant interaction HT * uPA/PAI-1 manifests itself in the figure in that the hazard reduction attributable to HT is not affected by uPA/PAI-1.

**Table 9: Multivariate Cox model (DFS) including interaction of uPA/PAI-1 with adjuvant treatment in primary**

| **Significant Factors** | **Coding for interpretation of β** | **Final model** | | |
|---|---|---|---|---|
| | | p-value | β | Hazard ratio (95 % Confidence interval) |
| **Involved lymph nodes** | fractional rank | < 0.001 | 2.47 | |
| | 1 node vs. node-negative | | | 2.2 (2.0 - 2.3) ^{a} |
| | 4 nodes vs. node-negative | | | 3.7 (3.3 - 4.2) ^{a} |
| | 10 nodes vs. node-negative | | | 5.3 (4.5 - 6.2) ^{a} |
| **Age** | squared fractional rank | < 0.001 | 1.29 | 3.65 (1.73 - 7.70) ^{b} |
| | fractional rank | < 0.001 | -1.94 | 0.14 (0,07 - 0,31)^{b} |
| **Tumor stage** | rest (1) vs. pT1 (0) | < 0.001 | 0.30 | 1.35 (1.19 - 1.54) |
| | rest (1) vs. pT1 / pT2 (0) | 0.008 | 0.22 | 1.24 (1.06 - 1.46), |
| **Progesterone *receptor*** | fractional rank | < 0.001 | -0.42 | 0.66 (0.55 - 0.80) ^{b} |
| **uPA/PAI-1** | high (1) vs. low(0) | < 0.001 | 0.70 | 2.01 (1.77 -2.28) |
| **Adjuvant chemotherapy** | yes (1) vs. no (0) | < 0.001 | -0.37 | 0.69 (0.56 - 0.85) |
| **Adjuvant endocrine therapy** | yes (1) vs. no (0) | < 0.001 | -0.38 | 0.68 (0.56 - 0.82) |
| **Interaction: "chemotherapy*uPA/PAI-1"** | both I vs. either or both 0 | 0.003 | -0.38 | 0.68 (0.53 - 0.88) |

| | | | | |
|---|---|---|---|---|
| ^{a} Hazard ratio for patients with 1,4, 10 positive nodes compared to node negative patients, CI approximate due to fractional ranks ^{b} Hazard for fractional rank = 1 compared to fractional rank = 0 | | | | |

### 6.2.6. DFS INCLUDING UPA/PAI-1 AND THEIR INTERACTIONS WITH THERAPY IN PATIENTS WITH 0-3 INVOLVED NODES

Separate analysis of the predictive value of uPA/PAI-1 in node-negative patients was not feasible, since less than about 5 % of patients in either subgroup received HT or CT. It is nonetheless of clinical interest to consider the group of patients with 0-3 involved nodes. Table 10 shows the proportional hazards analysis for DFS, which was stratified by data set as in Table 9 and which includes the same factors. Analysis was stratified by center: 50 patients were censored before first event in stratum, 800 events total. Stages of analysis are the same as in Table 9. One patient out of 2593 was excluded due to missing information on adjuvant chemotherapy.

In this subgroup of patients with 0-3 involved nodes, ranked Estrogen receptor is significant, higher values being associated with higher HR, while ranked Progesterone receptor is associated with lower HR. For tumor stage, only the distinction pT1 vs. all others is significant, but not pT1/pT2 vs. the rest. The HRs for age imply that young age is an even more strongly unfavorable factor in this subgroup than in all patients.

In patients with 0-3 affected lymph nodes, the effects involving therapy and uPA/PAI-1 are qualitatively and even quantitatively very close to those seen in the analysis of all patients. The hazard associated with high uPA/PAI-1 is slightly greater; adjuvant endocrine therapy has about the same benefit as in all patients; and the interaction between adjuvant chemotherapy and uPA/PAI-1 is similar; see FIG. 7.

**Table 10: Multivariate Cox model (DFS) including interaction of uPA/PAI-1 with adjuvant treatment in breast cancer patients with 0-3 involved axillary nodes (n=2592).**

| **Significant Factors^{b}** | **Coding for interpretation of β** | **Final model** | | |
|---|---|---|---|---|
| | | p-value | β | Hazard ratio ^{e} (95 % Confidence interval) |
| **Involved lymph nodes** | fractional rank | < 0.001 | 2.37 | |
| | 1 node vs. node-negative | | | 2.10 (1.8-2.4) ^{d} |
| | 2 nodes vs. node-negative | | | 2.70 (2.2-3.3) ^{d} |
| | 3 nodes vs. node-negative | | | 3.19 (2.5-4.0) ^{d} |
| **Age** | squared fractional rank | < 0.001 | 1.87 | 6.48 (2.52 -16.66) |
| | fractional rank | < 0.001 | -2.63 | 0.07 (0.03 - 0.19) |
| **Tumor stage** | rest (1) vs. pT1 (0) | < 0.001 | 0.35 | 1.42 (1.23 -1.65) |
| **Progesterone receptor** | fractional rank | < 0.001 | -0.58 | 0.56 (0.42 - 0.75) |
| **Estrogen receptor** | fractional rank | 0.008 | | 0.43 1.53 (1.12 - 2.10) |
| **UPA/PAI-1** | high (1) vs. low(0) | < 0.001 | 0.79 | 2.21 (1.88 -2.59) |
| **Adjuvant chemotherapy** | yes (1) vs. no (0) | 0.034 | -0.33 | 0.72 (0.53 - 0.98) |
| **Adjuvant endocrine therapy** | yes (1) vs. no (0) | 0.005 | -0.41 | 0.66 (0.48 -0.90) |
| **Interaction "chemotherapy*uPA/PAI-1"** | both 1 vs. either or both 0 | 0.041 | -0.36 | 0.70 (0.50 - 0.98) |

| | | | | |
|---|---|---|---|---|
| ^{d} Hazard ratio for patients with 1,2, 3 positive nodes compared to node negative patients, CI approximate due to fractional ranks ^{e} Hazard for fractional rank =1 compared to fractional rank = 0 | | | | |

### 6.2.7. DFS INCLUDING UPA/PAI-1 AND THEIR INTERACTIONS IN PATIENTS WITH FOUR OR MORE INVOLVED NODES

In patients with four or more involved axillary lymph nodes, the adjuvant therapy percentages are as follows: with low uPA/PAI-1 (n = 398, 5-year relapse rate 56 %), 27 % were treated by adjuvant endocrine therapy, and 36 % by chemotherapy. With high uPA/PAI-1 (n = 388, 5-year relapse rate 72 %), these percentages are slightly lower at 26 % and 29 %, respectively. The results of a Cox analysis performed for this subgroup are reported in Table 11. The factors were included as in the previous models; the analysis was again stratified by stratified by center: 7 patients censored before first event in stratum, 501 events total. Coding of significant factors were done according to Section 6.1. Stages of analysis are the same as in Table 9. Two patients were excluded due to missing information on adjuvant chemotherapy.

In these patients, it is noteworthy that uPA/PAI-1 has an enormous impact (b = log HR = 3.02), but there is also a large negative interaction of uPA/PAI-1 with lymph nodes (b = log HR = -2.79). There is also an (apparently) very high hazard (b = log HR = 5.36) associated with lymph nodes within this subgroup of patients with 4 or more affected nodes, but this number is partly an artifact of the representation in fractional ranks. To facilitate interpretation of this HR, as well as the interaction of lymph nodes with uPA/PAI-1, we compare the hazard for 10 vs. 4 affected nodes. For patients with low uPA/PAI-1, the HR of patients with 10 affected nodes is about twice as high as for 4 affected nodes, as seen in Table 11. (If this mere doubling of risk going from 4 to 10 nodes seems too moderate in view of b = 5.36, keep in mind that the fractional rank for lymph nodes for a patient with 4 nodes is already quite high, about 0.78.) In contrast, for patients with high uPA/PAI-1, the interaction means that the HR of patients with 10 affected nodes is only about 1.5 times that of patients with 4 affected nodes. Summarizing, the results (including interaction of lymph nodes with uPA/PAI-1) imply that the number of affected nodes even above 4 is important, but more so for low uPA/PAI-1 than for high uPA/PAI-1. In patients with >4 affected lymph nodes, the benefits of CT and HT and their relation to uPA/PAI-1 are again qualitatively and even quantitatively very close to those seen in the analysis of all patients and in the group with 0-3 affected nodes; see FIG. 7.

**Table 11: Multivariate Cox model (DFS) including interaction of uPA/PAI-1 with adjuvant treatment in breast cancer patients with 4 or more involved axillary nodes (n=784).**

| **Significant Factors** | **Coding for interpretation of β** | | **Final** | **model** |
|---|---|---|---|---|
| | | p-value | β | Hazard ratio^{b} (95 % Confidence interval) |
| **Involved lymph nodes** | 10 nodes vs. 4 nodes | < 0.001 | 5.36 | 2.1 (1.6 - 2.8) ^{a} |
| **Age** | fractional rank | 0.002 | -0.62 | 0.54 (0.36 - 0.80) |
| **Tumor stage** | rest (1) vs. pT1 / pT2 (0) | 0.001 | 0.35 | 1.41 (1.16 -1.73) |
| **Progesterone receptor** | fractional rank | 0.001 | -0.54 | 0.59 (0.43 - 0.80) |
| **uPA/PAI-1** | high (1) vs. low(0) | 0.013 | 3.02 | 20.5 (19 - 220) |
| **Adjuvant chemotherapy** | yes (1) vs. no (0) | 0.034 | -0.34 | 0.71 (0.52 - 0.97) |
| **Adjuvant endocrine therapy** | yes (1) vs. no (0) | 0.002 | -0.39 | 0.68 (0.53 - 0.87) |
| **Interaction "chemotherapy*uPA/PAI-1"** | both 1 vs. either or both 0 | 0.040 | -0.42 | 0.66 (0.44 - 0.98) |
| **Interaction "involved lymph nodes *uPA/PAI-1"** | if low uPA/PAI-1: zero | 0.039 | -2.79 | 0.061 (0.004 - 0.87) |
| | if high uPA/PAI-1: fractional rank of nodes | | | |

| | | | | |
|---|---|---|---|---|
| ^{a} Hazard ratio for patients with 10 positive nodes compared to patients with 4 positive nodes, confidence interval approximate due to fractional ranks ^{b} Hazard for fractional rank = 1 compared to fractional rank = 0 | | | | |

### 6.2.8. BENEFITS OF CT AND HT FOR DFS IN SUBGROUPS ACCORDING TO UPA/PAI-1

The effect of uPA/PAI-1 on response to therapy is also seen by constructing separate Cox models for high and low uPA/PAI-1 (again stratified by data set). In all patients, the HR is 0.68 due to CT and 0.74 due to HT according to a multivariate model for the low-uPA/PAI-1 subgroup. Within the multivariate model for the high-uPA/PAI-1 subgroup, the corresponding HRs are 0.49 due to CT and 0.63 due to HT. (In terms of log HR, the difference in HR for CT between low and high uPA/PAI-1 is about three standard errors, whereas for HT the corresponding difference is only one-third of a standard error.) Hence, these models are consistent with the tendency for more relative benefit due to CT in patients with high uPA/PAI-1 than in patients with low uPA/PAI-1 - after controlling for other factors.

Cox models were also performed separately for high and low uPA/PAI-1 patients in the subgroup of patients with 0-3 affected lymph nodes. In the low-uPA/PAI-1 group (n = 1418, 5-year relapse rate 20 %, 9 % receiving HT, 17 % receiving CT), it turns out that neither of the adjuvant therapy forms are significant: the 95 % CI for the HR of CT is 0.60 - 1.22, and for HT it is 0.59 - 1.44. Due to statistical uncertainty, in this subgroup a low to moderate benefit of either therapy is not ruled out. In contrast, in the high-uPA/PAI-1 subgroup (n = 1174, 5 -year relapse rate 38 %, 10 % receiving HT, 19 % receiving CT), both adjuvant therapy forms are significant and strong with a HR of 0.51 (0.33 - 0.78), HT approximately halves the hazard); the benefit of CT appears to be even stronger with a HR of 0.43 (95 % CI, 0.31 - 0.59). Comparing the result in these subgroups with the interaction analysis for 0-3 nodes reported above, the detection of a significant interaction CT * uPA/PAI-1 manifests itself in the uPA/PAI-1 subgroups as distinctly different HRs with non-intersecting confidence intervals. In the case of HT, the 95 % CI for the HRs in the two risk groups overlap substantially, and this is consistent with the lack of a significant interaction.

In patients with 4 or more affected nodes, a separate Cox regression (2-stage model) for the low-uPA/PAI-1 subgroup shows that these patients benefit significantly from adjuvant HT with a HR of 0.62 (95% CI, 0.43 - 0.90) and apparently also from CT with a HR of 0.70 (95% CI, 0.49 - 0.98). Lymph nodes are a very strong factor in this group (log HR = 5.68). The benefit from CT derived from the regression model for the group with 4 or more nodes and high uPA/PAI-1 is HR 0.60 (95% CI, 0.44 - 0.81). For HT the HR is 0.68 (95% CI, 0.49 - 0.95). Lymph nodes are weaker in this model (log HR = 2.43). These results taken together are consistent with the full model with interactions for patients with 4 or more nodes reported above.

### 6.3. DISCUSSION

The present inventors demonstrated that the particular combination, uPA/PAI-1 (both low vs. either or both high), achieves clinically relevant risk group discrimination over and above that provided by either factor alone, particularly in node-negative breast cancer. Furthermore, the present invention provide evidence for a benefit from adjuvant systemic therapy in high-risk patients as defined by uPA/PAI-1.

The plasminogen activator system plays an important role in tumor invasion and metastasis (Andreasen et al., 1997, Int J Cancer 72:1-22; Schmitt et al., 1997, Thromb Haemost 78:285-96; Stephens et al., 1998, Breast Cancer Res Treat 52:99-111). Patients with lymph node-negative breast cancer who are at risk for disease recurrence (high-risk patients) can be identified by the levels of uPA and PAI-1 in their primary tumor. About 45% of patients with lymph node-negative breast cancer belong to this high-risk group as defined by high levels of uPA and/or PAI-1 in their primary tumor. Harbeck et al., 1999, Br J Cancer 80: 419-26. Low-risk patients with lymph node-negative breast cancer have low levels of both uPA and PAI-1 in their tumor. This low-risk group, about 55% of all patients with lymph node-negative breast cancer, has an excellent prognosis, with a probability of relapse after 5 years of less than 5%. Harbeck et al., 1999, Br J Cancer 80: 419-26. Thus, there is little reason to generally recommend adjuvant chemotherapy to this group (Thomssen et al., 2000, Eur J Cancer 36:293-8; Hayes et al., 2000, Arbiter. Eur J Cancer 36:302-6), although, in an individual therapy decision, the patient's opinions on life-quality choices need to be considered. Ravdin et al., 1998, J Clin Oncol 16:515-21.

The ELISAs for uPA and PAI-1 are robust enough for clinical routine use, and international quality assurance is guaranteed. Sweep et al., 1998, Br J Cancer 78: 1434-1441. For testing, a minimum of 100 µg tumor tissue (corresponding to about 1 µg protein extract) is sufficient. Hence, the ELISAs can also be applied to extracts prepared from core biopsy specimens or cryostat sections. The optimized cutoffs for the assays used here are stable over time, correspond well to those found by other researchers using the same biochemical assays, (Foekens et al., 1994, J Clin Oncology 12:1648-1658) and have recently been validated in a multi-center prospective trial, Jänicke et al., 2001, J Natl Cancer Inst 93: 913-920. In contrast, no consistent clinically relevant data have been generated applying immunohistochemistry (IHC) or other techniques for determination of uPA and PAI-1 protein expression in breast carcinoma tissue. A recent IHC study showed that expression of uPA and PAI-1 in stromal fibroblasts is of more clinical relevance than expression in the tumor cells themselves, at least for the antibodies used. Dublin et al., 2000, Am J Pathol 157: 1219-1227. Such tissue heterogeneity with regard to expression of uPA and PAI-1 in different cell types is well accounted for using the ELISA procedure.

The fact that there is no contradictory evidence on the prognostic impact of uPA and PAI-1 in breast cancer is quite unique for any tumor biological factor, in particular given the fact that the data have been generated under a variety of demographic conditions (Europe, USA, and Japan). In a small node-negative collective without adjuvant systemic therapy, CART analysis shows that the combination of uPA and PAI-1 is superior to established prognostic factors with regard to selection of low-risk patients. It also outperforms other tumor biological factors such as HER2 protein overexpression, cathepsin D, p53, S-phase, MIB1 or DNA ploidy. Harbeck et al., 1999, Br J Cancer 80: 419-426. Even HER2 gene amplification as determined by FISH, which is also a strong prognostic factor in node-negative breast cancer, is complementary, though weaker than uPA/PAI-1, for risk-group selection in node-negative breast cancer.

The present invention described the great clinical value in testing both uPA and PAI-1 levels. In a collective of 269 node-negative patients without adjuvant systemic therapy, the condition "either or both high" identifies with high sensitivity those patients who are at high risk of relapse while still preserving a substantial, clinically relevant low-risk group. Thus, this combination captures and effectively dichotomizes the essential information obtained by the two factors. The significant improvement in risk discrimination (compared to either factor taken separately) is remarkable.

In the present invention, an interaction of uPA and PAI-1 was detected both using the proportional hazards assumption and using a time-varying model. The impact of uPA and PAI-1 can be described parsimoniously by the particular combination uPA/PAI-1. The time-variation implies that the relapses in the high-risk group will tend to occur within the first 3-4 years. Thus, not measuring one of these two factors might mean missing patients at high risk for subsequent systemic disease, and in particular those at risk for *early* relapse. Even within risk-groups defined by established prognostic factors, the combination uPA/PAI-1 enables significant risk-group assessment (Figure 5). Node-negative breast cancer patients with low levels of both PAI-1 and uPA in their primary tumor, comprising more than half of node negative patients, have an excellent 5-year DFS of more than 90 % (Figure 4). In contrast, those patients with high levels of either or both factors have a 5-year DFS comparable to that of patients with several involved lymph nodes (Figure 4).

Testing of both uPA and PAI-1 provides a valuable basis for patient counseling in node-negative breast cancer both for low and for high-risk patients. Some of these patients are willing to undergo systemic treatment for even a small benefit probability. Ravdin et al., 1998, J Clin Oncol 16: 515-521. However, other patients or their physicians will express a preference for a no-treatment option, in particular with regard to chemotherapy. The low-risk group identified by uPA/PAI-1 is substantially larger than that characterized by the St. Gallen criteria, (Goldhirsch et al., 1998, J Natl Cancer Inst 90:1601-1608) and thus much closer to the actual 70 % of node-negative patients cured by loco-regional treatment alone, (Clark et al., 1988, Semin Oncol 15:20-25) they are candidates for being spared the burden of adjuvant chemotherapy. This invention supports the potential value of uPA and PAI-1 measurements to define those node-negative patients who are clearly at high risk and for whom adjuvant systemic treatment would be strongly recommended.

The present invention discloses a benefit from *adjuvant* chemotherapy and/or endocrine therapy in patients with high uPA/PAI-1. In the patient collective as a whole, the univariate prognostic impact of uPA/PAI-1 on DFS was substantial in patients *without* adjuvant systemic therapy (Figure 6), underlining the strong association of uPA and PAI-1 with an aggressive tumor phenotype leading to invasion and metastasis. However, this prognostic strength is diminished in patients who received adjuvant systemic therapy, suggesting a benefit from adjuvant systemic therapy in this high-risk group, at least for DFS (Figure 6). The present invention shows that both uPA and PAI-1 are strong and significant even within the subgroup of node-positive patients, the majority of whom did not receive adjuvant systemic therapy.

uPA and PAI-1 are the only novel tumor biological factors so far which satisfy all of the strict criteria that may be used routinely in clinical settings. Nonetheless, the present invention shows for the prognostic value of uPA and PAI-1 as single factors measured by robust and quality assured ELISAs. The present invention also shows that the combination of both factors is superior to either factor taken alone and outperforms established prognostic factors with regard to risk-group stratification, in particular in node-negative breast cancer. Moreover, the present invention shows that high-risk patients according to uPA/PAI-1 benefit from adjuvant systemic therapy.

This invention shows that the clinical relevance of these two factors is greatest when used in combination and that the combination uPA/PAI-1 supports risk-adapted individualized therapeutic strategies in the adjuvant setting. Furthermore, this invention demonstrates that uPA and PAI-1 have not only a clinically relevant prognostic impact, but also a predictive impact in primary breast cancer.

For factors that do not strongly correlate with treatment decisions, the problem of confounding can be reduced by various methods, in particular by appropriate use of multivariate analysis and stratification. Since (in contrast to Estrogen receptor and Progesterone receptor) these requirements are satisfied rather well by uPA and PAI-1, the results disclosed by this invention should indeed reflect the predictive properties of uPA/PAI-1. An important step in "de-convoluting" the confounding factors in retrospective data is to introduce a multivariate statistical scoring model using as much of the information as possible in the other variables. A good scoring model will reduce the unexplained variation in the data and improve the chances of seeing interactions if they are present. Consequently, in this invention, a strategy of avoiding the use of cutoffs wherever possible, i.e., by representing most of the measurements as continuous variables, was applied. The only exception to the strategy of continuous variables were the factors uPA and PAI-1 themselves for which previously optimized cut-offs validated in a prospective multi-center trial were applied (Jänicke et al., 2001, Int. J. Natl. Cancer Inst., 93: 913-920; Harbeck et al., 1999, Breast Cancer Res. Treat., 54:147-157).

The results confirm that uPA/PAI-1 have a significant impact on patient outcome but also provides additional evidence supporting their use in the clinic by demonstrating how effects of adjuvant systemic therapy differ in patients classified according to uPA/PAI-1. As illustrated in FIG. 7, primary breast cancer patients with low uPA/PAI-1 generally benefit from adjuvant endocrine and chemotherapy. However, the benefits of chemotherapy (but not endocrine therapy) are strongly enhanced in patients with high uPA/PAI-1. It is important to note that patients with high uPA/PAI-1 also benefit from adjuvant endocrine therapy, even though adjuvant chemotherapy has a greater beneficial impact on their DFS.

Node-negative patients with low uPA/PAI-1 have a very low risk of relapse per se. The present invention found that endocrine therapy benefit low uPA/PAI-1 patients even with 0-3 affected nodes. Node-negative patients with low uPA/PAI-1 (but not those with high uPA/PAI-1) may be candidates for being spared the burden of adjuvant chemotherapy, but still could benefit from endocrine therapy if indicated - taking into account the known side effects of chemotherapy and the preventive benefits of endocrine therapy.

Retrospective analyses in advanced and metastatic breast cancer showed decreased response to palliative endocrine therapy in patients with high uPA or PAI-1 levels in primary tumor tissue compared to patients with low levels (Jänicke et al., 1994, "Urokinase (uPA) and PAI-1 as selection criteria for adjuvant chemotherapy in axillary node-negative breast cancer patients, " in Prospects in Diagnosis and Treatment of Cancer, Schmitt et al. eds: 207-218; Foekens et al., 1995, J. Natl. Cancer Inst. 87: 751-756). This should not be regarded as a contradiction to results of this invention, which was obtained in the adjuvant setting, but can be understood taking the underlying tumor biology into account. High levels of uPA and PAI-1 do reflect an aggressive phenotype which may be overcome or suppressed by early systemic therapy as in the adjuvant setting but may be far too advanced for response to palliative therapy at a later stage.

The intended clinical application of the present invention is to exploit fully the risk assessment information provided by uPA/PAT-1 in the context of clinical decision making in primary breast cancer. The rationale is not limited to that of finding a very low-risk group who could be spared systemic treatment altogether, but rather to understand on the basis of the currently available evidence - including uPA and PAI-1 measurements - which treatment options are benefiting which patients. In conclusion, breast cancer patients with high uPA/PAI-1 have a more aggressive disease stage than conventional factors would otherwise lead the physician to believe. However, the present invention provide evidence that the DFS disadvantage due to their more aggressive disease phenotype can be largely counteracted by adjuvant endocrine therapy and in particular by adjuvant chemotherapy as illustrated in Figure 7. Although uPA/PAI-1 are not the only variables that should be taken into account for therapy decisions, the present invention suggests that a significant and substantial improvement in decision support will be achievable by testing breast cancer patients for uPA and PAI-1. Hence, considering the underlying tumor biology and our finding that high-risk patients according to uPA/PAI-1 benefit from conventional adjuvant systemic therapy, particularly from chemotherapy, it is all the more promising to combine novel therapeutics targeting the plasminogen activation system with such conventional systemic therapy.

Many modifications and variations of this invention can be made without departing from its scope, as will be apparent to those skilled in the art. The specific embodiments described herein are offered by way of example only, and the invention is to be limited only by the terms of the appended claims, along with the full scope of equivalents to which such claims are entitled.

## Claims

1. A method for selecting from two or more treatment regimens, a treatment regimen having the highest expected benefit to a patient with primary breast cancer or, a method for predicting an expected benefit in a comparable population of breast cancer patients by selecting from two or more treatment regimens, a treatment regimen having the highest expected benefit, said patient having primary tumor tissue, said method comprising:
(a) measuring the level of uPA and the level of PAI-1 or mRNA encoding uPA and mRNA encoding PAI-1 in a sample of said primary tumor tissue of said patient;
(b) classifying said patient as low risk if the level of uPA is lower than a uPA cut-off value of at least the 55^{th} percentile and at most the 75^{th} percentile of normalized uPA levels in a randomized population of breast cancer patients and the level of PAI-1 is lower than a PAI-1 cut-off value of at least the 61^{st} percentile and at most the 81^{st} percentile of normalized PAI-1 levels in a randomized population of breast cancer patients, or as high risk if either the level of uPA is higher than the uPA cut-off value or the level of PAI-1 is higher than the PAI-1 cut-off value;
(c) if said patient is classified as low risk in step (b), selecting a treatment regimen from said two or more treatment regimens that results in the highest expected benefit in a comparable population of low risk cancer patients; and
(d) if said patient is classified as high risk in step (b), selecting a treatment regimen from said two or more treatment regimens that results in the highest expected benefit in a comparable population of high risk cancer patients.

2. The method of claim 1 wherein if said patient is classified as high risk, the selected treatment regimen is selected from chemotherapy, adjuvant chemotherapy, adjuvant CMF chemotherapy, adjuvant non-CMF chemotherapy, adjuvant anthracyclin-containing chemotherapy, adjuvant taxane-containing chemotherapy, adjuvant endocrine therapy, radiation therapy, gene therapy, immunotherapy or tumor-biological therapy.

3. The method of claim 1 wherein said mRNA encoding uPA and mRNA encoding PAI-1 are measured by RT-PCR amplification.

4. The method of claim 1 wherein said comparable population is defined by nodal status, number of nodes affected, tumor size, tumor grade, patient's age, hormone receptor status, and menopausal status.

5. The method of claim 1 wherein said patient is node negative.

6. The method of claim 1 wherein said patient has no more than 3 affected lymph nodes.

7. The method of claim 1 wherein, if said patient is classified as low risk, the selected treatment regimen is selected from no treatment, radiation therapy, or adjuvant endocrine therapy.

8. The method of claim 1 wherein said sample is collected by core needle biopsy or body fluid aspiration.

9. The method of claim 1 wherein said level of uPA and said level of PAI-1 are measured by *in situ* quantitation.

10. The method of claim 1 wherein said level of uPA and said level of PAI-1 are measured using immunofluorescence or immunoelectron microscopy.

11. The method of claim 10 wherein said level of uPA and said level of PAI-1 are measured quantitatively by counting the number of grains of label on said sample.

12. The method of claim 1 wherein said sample is a histological specimen from said patient.

13. The method of claim 12 wherein said histological specimen is a tissue section embedded in paraffin.

14. The method of claim 13 wherein said level of uPA and said level of PAI-1 are measured by contacting said tissue section with uPA antibodies and PAI-1 antibodies.

15. The method of claim 1 wherein said treatment regimen for high risk patient is administration of bisphosphonate drugs to the patient.

## Patentansprüche

1. Verfahren zum Auswählen einer Behandlungsmethode, die den höchsten zu erwartenden Nutzen für einen Patienten mit primärem Brustkrebs hat, aus zwei oder mehr Behandlungsmethoden oder Verfahren zur Vorhersage eines zu erwartenden Nutzens in einer vergleichbaren Population von Brustkrebspatienten durch Auswahl einer Behandlungsmethode, die den höchsten zu erwartenden Nutzen hat, aus zwei oder mehr Behandlungsmethoden, wobei der Patient primäres Tumorgewebe aufweist, wobei die Methode:
(a) Messen des uPA-Levels und des PAI-1-Levels oder mRNA, welche uPA kodiert, und mRNA, welche PAI-1 kodiert, in einer Probe des primären Tumorgewebes des Patienten;
(b) Klassifizieren des Patienten als risikoarm, wenn das uPA-Level niedriger als ein uPA-Schwellenwert von mindestens dem 55. Perzentil und höchstens dem 75. Perzentil des normalisierten uPA-Levels in einer randomisierten Population von Brustkrebspatienten ist und das PAI-1-Level niedriger als ein PAI-1-Schwellenwert von mindestens dem 61. Perzentil und höchstens dem 81. Perzentil des normalisierten PAI-1-Levels in einer randomisierten Population von Brustkrebspatienten ist oder als risikoreich, wenn entweder das uPA-Level höher als der uPA-Schwellenwert ist oder das PAI-1-Level höher als der PAI-1-Schwellenwert ist;
(c) Auswahl einer Behandlungsmethode, die den höchsten zu erwartenden Nutzen in einer vergleichbaren Population von Krebspatienten mit geringem Risiko bringt, aus den zwei oder mehr Behandlungsmethoden, wenn der Patient in Schritt (b) als risikoarm klassifiziert ist; und
(d) Auswahl einer Behandlungsmethode, die den höchsten zu erwartenden Nutzen in einer vergleichbaren Population von Krebspatienten mit hohem Risiko bringt, aus den zwei oder mehr Behandlungsmethoden, wenn der Patient in Schritt (b) als risikoreich klassifiziert ist;
umfasst.

2. Verfahren nach Anspruch 1, wobei, wenn der Patient als risikoreich klassifiziert ist, die ausgewählte Behandlungsmethode aus Chemotherapie, adjuvante Chemotherapie, adjuvante CMF-Chemotherapie, adjuvante nicht-CMF-Chemotherapie, adjuvante Anthracyclin-enthaltende Chemotherapie, adjuvante Taxan-enthaltende Chemotherapie, adjuvante endokrine Therapie, Strahlentherapie, Gentherapie, Immuntherapie oder tumorbiologische Therapie ausgewählt ist.

3. Verfahren nach Anspruch 1, wobei die mRNA, welche uPA kodiert, und die mRNA, welche PAI-1 kodiert, durch RT-PCR-Amplifikation gemessen werden.

4. Verfahren nach Anspruch 1, wobei die vergleichbare Population durch den Nodalstatus, Anzahl der betroffenen Lymphknoten, Tumorgröße, Tumorgrad, Alter des Patienten, Hormonrezeptorstatus und Menopausenstatus definiert ist.

5. Verfahren nach Anspruch 1, wobei der Patient nodal-negativ ist.

6. Verfahren nach Anspruch 1, wobei der Patient nicht mehr als 3 betroffene Lymphknoten hat.

7. Verfahren nach Anspruch 1, wobei, wenn der Patient als risikoarm klassifiziert ist, die ausgewählte Behandlungsmethode aus keine Behandlung, Strahlentherapie oder adjuvante endokrine Therapie ausgewählt ist.

8. Verfahren nach Anspruch 1, wobei die Probe durch "core needle"-Biopsie oder Körperflüssigkeitsaspiration gewonnen wird.

9. Verfahren nach Anspruch 1, wobei das uPA-Level und das PAI-1-Level durch *in situ* Quantifizierung gemessen werden.

10. Verfahren nach Anspruch 1, wobei das uPA-Level und das PAI-1-Level unter Verwendung von Immunfluoreszenz oder Immunelektronenmikroskopie gemessen werden.

11. Verfahren nach Anspruch 10, wobei das uPA-Level und das PAI-1-Level quantitativ durch Zählen der Anzahl der Labelkörner auf der Probe gemessen werden.

12. Verfahren nach Anspruch 1, wobei die Probe eine histologische Probe des Patienten ist.

13. Verfahren nach Anspruch 12, wobei die histologische Probe ein in Paraffin eingebetteter Gewebsschnitt ist.

14. Verfahren nach Anspruch 13, wobei das uPA-Level und das PAI-1-Level durch Inkontaktbringen des Gewebeschnittes mit uPA-Antikörpern und PAI-1-Antikörpern gemessen werden.

15. Verfahren nach Anspruch 1, wobei die Behandlungsmethode für Patienten mit hohem Risiko die Verabreichung von Bisphosphonat-Wirkstoffen an diese Patienten ist.

## Revendications

1. Un procédé pour sélectionner parmi deux ou plus régimes de traitement un régime de traitement ayant le bénéfice attendu le plus élevé pour un patient atteint de cancer du sein primaire ou un procédé pour prédire un bénéfice attendu dans une population de patients atteints de cancer du sein comparable en sélectionnant parmi deux ou plus régimes de traitement un régime de traitement ayant le bénéfice attendu le plus élevé, le patient ayant tissu tumoral primaire, le procédé comprenant:
(a) mesurer le niveau d'uPA et le niveau de PAI-1 ou ARNm encodant uPA et ARNm encodant PAI-1 dans un échantillon du tissu tumoral primaire du patient;
(b) classifier le patient comme basse risque si le niveau d'uPA est inférieur à une valeur limite d'uPA du au minimum 55^{ème} centile et du au maximum 75^{ème} centile de niveaux d'uPA normalisés dans une population de patients atteints de cancer du sein randomisée et le niveau de PAI-1 est inférieur à une valeur limite de PAI-1 du au minimum 61^{ème} centile et du au maximum 81^{ème} centile de niveaux de PAI-1 normalisés dans une population de patients atteints de cancer du sein randomisée ou comme haute risque si soit le niveau d'uPA est supérieur à la valeur limite d'uPA soit le niveau de PAI-1 est supérieur à la valeur limite de PAI-1;
(c) si le patient est classifié comme basse risque dans l'étape (b), sélectionner un régime de traitement parmi les deux ou plus régimes de traitement qui résulte dans le bénéfice attendu le plus élevé dans une population de patients atteints de cancer de basse risque comparable; et
(d) si le patient est classifié comme haute risque dans l'étape (b), sélectionner un régime de traitement parmi les deux ou plus régimes de traitement qui résulte dans le bénéfice attendu le plus élevé dans une population de patients atteints de cancer de haute risque comparable.

2. Le procédé de la revendication 1, où, si le patient est classifié comme haute risque, le régime de traitement sélectionné est sélectionné parmi chimiothérapie, chimiothérapie adjuvante, chimiothérapie CMF adjuvante, chimiothérapie non-CMF adjuvante, chimiothérapie adjuvante contenant anthracycline, chimiothérapie adjuvante contenant taxane, thérapie endocrine adjuvante, radiothérapie, thérapie génique, immunothérapie ou thérapie tumeur-biologique.

3. Le procédé de la revendication 1, où l'ARNm encodant uPA et l'ARNm encodant PAI-1 sont mesurés par amplification PCR-TR.

4. Le procédé de la revendication 1, où la population comparable est définie par l'état nodal, le nombre de noeuds concernés, la grandeur du tumeur, le grade de tumeur, l'âge du patient, l'état de récepteur d'hormone et l'état ménopausal.

5. Le procédé de la revendication 1, où le patient est noeud-négatif.

6. Le procédé de la revendication 1, où le patient n'a pas plus que 3 noeuds lymphatiques concernés.

7. Le procédé de la revendication 1, où, si le patient est classifié comme basse risque, le régime de traitement sélectionné est sélectionné parmi pas de traitement, radiothérapie ou thérapie endocrine adjuvante.

8. Le procédé de la revendication 1, où l'échantillon est prélevé par biopsie au trocart ou aspiration de fluide corporel.

9. Le procédé de la revendication 1, où le niveau d'uPA et le niveau de PAI-1 sont mesurés par quantification *in situ.*

10. Le procédé de la revendication 1, où le niveau d'uPA et le niveau de PAI-1 sont mesurés en utilisant immunofluorescence ou microscopie immunoélectronique.

11. Le procédé de la revendication 10, où le niveau d'uPA et le niveau de PAI-1 sont mesurés de manière quantitative en comptant le nombre de grains de label sur l'échantillon.

12. Le procédé de la revendication 1, où l'échantillon est un spécimen histologique du patient.

13. Le procédé de la revendication 12, où le spécimen histologique est une section de tissu inséré dans paraffine.

14. Le procédé de la revendication 13, où le niveau d'uPA et le niveau de PAI-1 sont mesurés en contactant la section de tissu avec des anticorps uPA et des anticorps PAI-1.

15. Le procédé de la revendication 1, où le régime de traitement pour patient à haute risque est l'administration de médicaments de bisphosphonate au patient.


### SEQUENCE LISTING

<110> AMERICAN DIAGNOSTICA, INC.
<120> METHODS FOR SELECTING TREATMENT REGIMENS AND PREDICTING OUTCOMES IN BREAST CANCER PATIENTS
<130> 9471-013-228
<140> To be assigned
   <141> Herewith
<150> 60/356,928
   <151> 2002-02-13
<150> 60/402,311
   <151> 2002-08-09
<160> 4
<170> FastSEQ for Windows Version 4.0
<210> 1
   <211> 1296
   <212> DNA
   <213> Homo Sapiens
<400> 1
   atgagagccc tgctggcgcg cctgcttctc tgcgtcctgg tcgtgagcga ctccaaaggc 60
   agcaatgaac ttcatcaagt tccatcgaac tgtgactgtc taaatggagg aacatgtgtg 120
   tccaacaagt acttctccaa cattcactgg tgcaactgcc caaagaaatt cggagggcag 180
   cactgtgaaa tagataagtc aaaaacctgc tatgagggga atggtcactt ttaccgagga 240
   aaggccagca ctgacaccat gggccggccc tgcctgccct ggaactctgc cactgtcctt 300
   cagcaaacgt accatgccca cagatctgat gctcttcagc tgggcctggg gaaacataat 360
   tactgcagga acccagacaa ccggaggcga ccctggtgct atgtgcaggt gggcctaaag 420
   ccgcttgtcc aagagtgcat ggtgcatgac tgcgcagatg gaaaaaagcc ctcctctcct 480
   ccagaagaat taaaatttca gtgtggccaa aagactctga ggccccgctt taagattatt 540
   gggggagaat tcaccaccat cgagaaccag ccctggtttg cggccatcta caggaggcac 600
   cgggggggct ctgtcaccta cgtgtgtgga ggcagcctca tcagcccttg ctgggtgatc 660
   agcgccacac actgcttcat tgattaccca aagaaggagg actacatcgt ctacctgggt 720
   cgctcaaggc ttaactccaa cacgcaaggg gagatgaagt ttgaggtgga aaacctcatc 780
   ctacacaagg actacagcgc tgacacgctt gctcaccaca atgacattgc cttgctgaag 840
   atccgttcca aggagggcag gtgtgcgcag ccatcccgga ctatacagac catctgcctg 900
   ccctcgatgt ataacgatcc ccagtttggc acaagctgtg agatcactgg ctttggaaaa 960
   gagaattcta ccgactatct ctatccggag cagctgaaaa tgactgttgt gaagctgatt 1020
   tcccaccggg agtgtcagca gccccactac tacggctctg aagtcaccac caaaatgctg 1080
   tgtgctgctg acccacagtg gaaaacagat tcctgccagg gagactcagg gggacccctc 1140
   gtctgttccc tccaaggccg catgactttg actggaattg tgagctgggg ccgtggatgt 1200
   gccctgaagg acaagccagg cgtctacacg agagtctcac acttcttacc ctggatccgc 1260
   agtcacacca aggaagagaa tggcctggcc ctctga 1296
<210> 2
   <211> 431
   <212> PRT
   <213> Homo Sapiens
<400> 2
<210> 3
   <211> 1209
   <212> DNA
   <213> Homo Sapiens
<400> 3
   atgcagatgt ctccagccct cacctgccta gtcctgggcc tggcccttgt ctttggtgaa 60
   gggtctgctg tgcaccatcc cccatcctac gtggcccacc tggcctcaga cttcggggtg 120
   agggtgtttc agcaggtggc gcaggcctcc aaggaccgca acgtggtttt ctcaccctat 180
   ggggtggcct cggtgttggc catgctccag ctgacaacag gaggagaaac ccagcagcag 240
   attcaagcag ctatgggatt caagattgat gacaagggca tggcccccgc cctccggcat 300
   ctgtacaagg agctcatggg gccatggaac aaggatgaga tcagcaccac agacgcgatc 360
   ttcgtccagc gggatctgaa gctggtccag ggcttcatgc cccacttctt caggctgttc 420
   cggagcacgg tcaagcaagt ggacttttca gaggtggaga gagccagatt catcatcaat 480
   gactgggtga agacacacac aaaaggtatg atcagcaact tgcttgggaa aggagccgtg 540
   gaccagctga cacggctggt gctggtgaat gccctctact tcaacggcca gtggaagact 600
   cccttccccg actccagcac ccaccgccgc ctcttccaca aatcagacgg cagcactgtc 660
   tctgtgccca tgatggctca gaccaacaag ttcaactata ctgagttcac cacgcccgat 720
   ggccattact acgacatcct ggaactgccc taccacgggg acaccctcag catgttcatt 780
   gctgcccctt atgaaaaaga ggtgcctctc tctgccctca ccaacattct gagtgcccag 840
   ctcatcagcc actggaaagg caacatgacc aggctgcccc gcctcctggt tctgcccaag 900
   ttctccctgg agactgaagt cgacctcagg aagcccctag agaacctggg aatgaccgac 960
   atgttcagac agtttcaggc tgacttcacg agtctttcag accaagagcc tctccacgtc 1020
   gcgcaggcgc tgcagaaagt gaagatcgag gtgaacgaga gtggcacggt ggcctcctca 1080
   tccacagctg tcatagtctc agcccgcatg gcccccgagg agatcatcat ggacagaccc 1140
   ttcctctttg tggtccggca caaccccaca ggaacagtcc ttttcatggg ccaagtgatg 1200
   gaaccctga                                                         1209
<210> 4
   <211> 402
   <212> PRT
   <213> Homo Sapiens
<400> 4